# EUROPÄISCHE PATENTANMELDUNG

(11) **EP 1 950 222 A1**
(43) Veröffentlichungstag der Anmeldung: **30.07.2008**
(21) Anmeldenummer: 07090010.5
(22) Anmeldetag: 26.01.2007
(51) Int. Cl.: C07K 14/47, A61K 38/17, A61K 39/00, G01N 33/68

(54) **Verfahren zum Nachweis von Antikörpern aus Körperflüssigkeiten durch eine Immunreaktion mit Glykoprotein 2 (GP2) aus zymogenen Granula des Pankreas zur Differentialdiagnose von entzündlichen Darmerkrankungen und chronischer Pankreatitis**

(71) Anmelder: GA Generic Assays GmbH, 15827 Dahlewitz (DE)
(72) Erfinder: Roggenbuck, Dirk, Dr., 15827 Dahlewitz (DE)
(74) Vertreter: Boeckh, Tobias

(57) **Zusammenfassung**

Die Erfindung betrifft ein Verfahren zum Nachweis von Antikörpern aus Körperflüssigkeiten durch eine Immunreaktion mit GP2 aus zymogenen Granula des Pankreas, dessen immunreaktive Sequenzen oder Analoga unter Ausschluss von Gewebeschnitten.

SEQUENCE LISTING

<110>  GA GENERIC ASSAYS GmbH

<120>  Verfahren zum Nachweis von Antikörpern aus Körperflüssigkeiten
       durch eine Immunreaktion mit Glykoprotein 2 (GP2) aus zymogenen
       Granula des Pankreas zur Differentialdiagnose von entzündlichen
       Darmerkrankungen und chronischer Pankreatitis

<130>  XII 114-07

<140>  07090010.5
<141>  2007-01-26

<160>  5

<170>  PatentIn version 3.3

<210>  1
<211>  527
<212>  PRT
<213>  Homo sapiens


<220>
<221>  MISC_FEATURE
<223>  Glykoprotein 2

<400>  1

Met Val Gly Ser Gly Leu Leu Trp Leu Ala Leu Val Ser Cys Ile Leu
1               5                   10                  15


Thr Gln Ala Ser Ala Val Gln Arg Gly Tyr Gly Asn Pro Ile Glu Ala
            20                  25                  30


Ser Ser Tyr Gly Leu Asp Leu Asp Cys Gly Ala Pro Gly Thr Pro Glu
        35                  40                  45


Ala His Val Cys Phe Asp Pro Cys Gln Asn Tyr Thr Leu Leu Asp Glu
    50                  55                  60


Pro Phe Arg Ser Thr Glu Asn Ser Ala Gly Ser Gln Gly Cys Asp Lys
65                  70                  75                  80


Asn Met Ser Gly Trp Tyr Arg Phe Val Gly Glu Gly Gly Val Arg Met
                85                  90                  95


Ser Glu Thr Cys Val Gln Val His Arg Cys Gln Thr Asp Ala Pro Met
            100                 105                 110


Trp Leu Asn Gly Thr His Pro Ala Leu Gly Asp Gly Ile Thr Asn His
        115                 120                 125


Thr Ala Cys Ala His Trp Ser Gly Asn Cys Cys Phe Trp Lys Thr Glu
    130                 135                 140


Val Leu Val Lys Ala Cys Pro Gly Gly Tyr His Val Tyr Arg Leu Glu
145                 150                 155                 160

Gly Thr Pro Trp Cys Asn Leu Arg Tyr Cys Thr Asp Pro Ser Thr Val
                165                 170                 175

Glu Asp Lys Cys Glu Lys Ala Cys Arg Pro Glu Glu Glu Cys Leu Ala
            180                 185                 190

Leu Asn Ser Thr Trp Gly Cys Phe Cys Arg Gln Asp Leu Asn Ser Ser
        195                 200                 205

Asp Val His Ser Leu Gln Pro Gln Leu Asp Cys Gly Pro Arg Glu Ile
    210                 215                 220

Lys Val Lys Val Asp Lys Cys Leu Leu Gly Gly Leu Gly Leu Gly Glu
225                 230                 235                 240

Glu Val Ile Ala Tyr Leu Arg Asp Pro Asn Cys Ser Ser Ile Leu Gln
                245                 250                 255

Thr Glu Glu Arg Asn Trp Val Ser Val Thr Ser Pro Val Gln Ala Ser
            260                 265                 270

Ala Cys Arg Asn Ile Leu Glu Arg Asn Gln Thr His Ala Ile Tyr Lys
        275                 280                 285

Asn Thr Leu Ser Leu Val Asn Asp Phe Ile Ile Arg Asp Thr Ile Leu
    290                 295                 300

Asn Ile Asn Phe Gln Cys Ala Tyr Pro Leu Asp Met Lys Val Ser Leu
305                 310                 315                 320

Gln Ala Ala Leu Gln Pro Ile Val Ser Ser Leu Asn Val Ser Val Asp
                325                 330                 335

Gly Asn Gly Glu Phe Ile Val Arg Met Ala Leu Phe Gln Asp Gln Asn
            340                 345                 350

Tyr Thr Asn Pro Tyr Glu Gly Asp Ala Val Glu Leu Ser Val Glu Ser
        355                 360                 365

Val Leu Tyr Val Gly Ala Ile Leu Glu Gln Gly Asp Thr Ser Arg Phe
    370                 375                 380

Asn Leu Val Leu Arg Asn Cys Tyr Ala Thr Pro Thr Glu Asp Lys Ala
385                 390                 395                 400

Asp Leu Val Lys Tyr Phe Ile Ile Arg Asn Ser Cys Ser Asn Gln Arg
                405                 410                 415

Asp Ser Thr Ile His Val Glu Glu Asn Gly Gln Ser Ser Glu Ser Arg
            420                 425                 430

Phe Ser Val Gln Met Phe Met Phe Ala Gly His Tyr Asp Leu Val Phe
        435                 440                 445

Leu His Cys Glu Ile His Leu Cys Asp Ser Leu Asn Glu Gln Cys Gln
    450                 455                 460

Pro Ser Cys Ser Arg Ser Gln Val Arg Ser Glu Val Pro Ala Ile Asp
465                 470                 475                 480

Leu Ala Arg Val Leu Asp Leu Gly Pro Ile Thr Arg Arg Gly Ala Gln
                485                 490                 495

Ser Pro Gly Val Met Asn Gly Thr Pro Ser Thr Ala Gly Phe Leu Val
            500                 505                 510

Ala Trp Pro Met Val Leu Leu Thr Val Leu Leu Ala Trp Leu Phe
        515                 520                 525

<210>  2
<211>  537
<212>  PRT
<213>  Homo sapiens

<220>
<221>  MISC_FEATURE
<223>  Glykoprotein 2

<400>  2

Met Pro His Leu Met Glu Arg Met Val Gly Ser Gly Leu Leu Trp Leu
1               5                   10                  15

Ala Leu Val Ser Cys Ile Leu Thr Gln Ala Ser Ala Val Gln Arg Gly
            20                  25                  30

Tyr Gly Asn Pro Ile Glu Ala Ser Ser Tyr Gly Leu Asp Leu Asp Cys
        35                  40                  45

Gly Ala Pro Gly Thr Pro Glu Ala His Val Cys Phe Asp Pro Cys Gln
    50                  55                  60

Asn Tyr Thr Leu Leu Asp Glu Pro Phe Arg Ser Thr Glu Asn Ser Ala
65                  70                  75                  80

Gly Ser Gln Gly Cys Asp Lys Asn Met Ser Gly Trp Tyr Arg Phe Val
                85                  90                  95

Gly Glu Gly Gly Val Arg Met Ser Glu Thr Cys Val Gln Val His Arg
            100                 105                 110

Cys Gln Thr Asp Ala Pro Met Trp Leu Asn Gly Thr His Pro Ala Leu
        115                 120                 125

Gly Asp Gly Ile Thr Asn His Thr Ala Cys Ala His Trp Ser Gly Asn
    130                 135                 140

Cys Cys Phe Trp Lys Thr Glu Val Leu Val Lys Ala Cys Pro Gly Gly
145                 150                 155                 160

Tyr His Val Tyr Arg Leu Glu Gly Thr Pro Trp Cys Asn Leu Arg Tyr
                165                 170                 175

Cys Thr Val Pro Arg Asp Pro Ser Thr Val Glu Asp Lys Cys Glu Lys
            180                 185                 190

Ala Cys Arg Pro Glu Glu Glu Cys Leu Ala Leu Asn Ser Thr Trp Gly
        195                 200                 205

Cys Phe Cys Arg Gln Asp Leu Asn Ser Ser Asp Val His Ser Leu Gln
    210                 215                 220

Pro Gln Leu Asp Cys Gly Pro Arg Glu Ile Lys Val Lys Val Asp Lys
225                 230                 235                 240

Cys Leu Leu Gly Gly Leu Gly Leu Gly Glu Glu Val Ile Ala Tyr Leu
                245                 250                 255

Arg Asp Pro Asn Cys Ser Ser Ile Leu Gln Thr Glu Glu Arg Asn Trp
            260                 265                 270

Val Ser Val Thr Ser Pro Val Gln Ala Ser Ala Cys Arg Asn Ile Leu
        275                 280                 285

Glu Arg Asn Gln Thr His Ala Ile Tyr Lys Asn Thr Leu Ser Leu Val
    290                 295                 300

Asn Asp Phe Ile Ile Arg Asp Thr Ile Leu Asn Ile Asn Phe Gln Cys
305                 310                 315                 320

Ala Tyr Pro Leu Asp Met Lys Val Ser Leu Gln Ala Ala Leu Gln Pro
                325                 330                 335

Ile Val Ser Ser Leu Asn Val Ser Val Asp Gly Asn Gly Glu Phe Ile
            340                 345                 350

Val Arg Met Ala Leu Phe Gln Asp Gln Asn Tyr Thr Asn Pro Tyr Glu
        355                 360                 365

Gly Asp Ala Val Glu Leu Ser Val Glu Ser Val Leu Tyr Val Gly Ala
    370                 375                 380

Ile Leu Glu Gln Gly Asp Thr Ser Arg Phe Asn Leu Val Leu Arg Asn
385                 390                 395                 400

Cys Tyr Ala Thr Pro Thr Glu Asp Lys Ala Asp Leu Val Lys Tyr Phe
                405                 410                 415

Ile Ile Arg Asn Ser Cys Ser Asn Gln Arg Asp Ser Thr Ile His Val
            420                 425                 430

Glu Glu Asn Gly Gln Ser Ser Glu Ser Arg Phe Ser Val Gln Met Phe
        435                 440                 445

Met Phe Ala Gly His Tyr Asp Leu Val Phe Leu His Cys Glu Ile His
    450                 455                 460

Leu Cys Asp Ser Leu Asn Glu Gln Cys Gln Pro Ser Cys Ser Arg Ser
465                 470                 475                 480

Gln Val Arg Ser Glu Val Pro Ala Ile Asp Leu Ala Arg Val Leu Asp
                485                 490                 495

Leu Gly Pro Ile Thr Arg Arg Gly Ala Gln Ser Pro Gly Val Met Asn
            500                 505                 510

Gly Thr Pro Ser Thr Ala Gly Phe Leu Val Ala Trp Pro Met Val Leu
        515                 520                 525

Leu Thr Val Leu Leu Ala Trp Leu Phe
    530                 535

<210>  3
<211>  534
<212>  PRT
<213>  Homo sapiens

<220>
<221>  MISC_FEATURE
<223>  Glykoprotein 2

<400>  3

Met Pro His Leu Met Glu Arg Met Val Gly Ser Gly Leu Leu Trp Leu
1               5                   10                  15

Ala Leu Val Ser Cys Ile Leu Thr Gln Ala Ser Ala Val Gln Arg Gly
            20                  25                  30

Tyr Gly Asn Pro Ile Glu Ala Ser Ser Tyr Gly Leu Asp Leu Asp Cys
        35                  40                  45

Gly Ala Pro Gly Thr Pro Glu Ala His Val Cys Phe Asp Pro Cys Gln
    50                  55                  60

Asn Tyr Thr Leu Leu Asp Glu Pro Phe Arg Ser Thr Glu Asn Ser Ala
65                  70                  75                  80

Gly Ser Gln Gly Cys Asp Lys Asn Met Ser Gly Trp Tyr Arg Phe Val
                85                  90                  95

Gly Glu Gly Gly Val Arg Met Ser Glu Thr Cys Val Gln Val His Arg
            100                 105                 110

Cys Gln Thr Asp Ala Pro Met Trp Leu Asn Gly Thr His Pro Ala Leu
        115                 120                 125

Gly Asp Gly Ile Thr Asn His Thr Ala Cys Ala His Trp Ser Gly Asn
    130                 135                 140

Cys Cys Phe Trp Lys Thr Glu Val Leu Val Lys Ala Cys Pro Gly Gly
145                 150                 155                 160

Tyr His Val Tyr Arg Leu Glu Gly Thr Pro Trp Cys Asn Leu Arg Tyr
                165                 170                 175

Cys Thr Asp Pro Ser Thr Val Glu Asp Lys Cys Glu Lys Ala Cys Arg
            180                 185                 190

Pro Glu Glu Glu Cys Leu Ala Leu Asn Ser Thr Trp Gly Cys Phe Cys
        195                 200                 205

Arg Gln Asp Leu Asn Ser Ser Asp Val His Ser Leu Gln Pro Gln Leu
    210                 215                 220

Asp Cys Gly Pro Arg Glu Ile Lys Val Lys Val Asp Lys Cys Leu Leu
225                 230                 235                 240

Gly Gly Leu Gly Leu Gly Glu Glu Val Ile Ala Tyr Leu Arg Asp Pro
                245                 250                 255

Asn Cys Ser Ser Ile Leu Gln Thr Glu Glu Arg Asn Trp Val Ser Val
            260                 265                 270

Thr Ser Pro Val Gln Ala Ser Ala Cys Arg Asn Ile Leu Glu Arg Asn
        275                 280                 285

Gln Thr His Ala Ile Tyr Lys Asn Thr Leu Ser Leu Val Asn Asp Phe
    290                 295                 300

Ile Ile Arg Asp Thr Ile Leu Asn Ile Asn Phe Gln Cys Ala Tyr Pro
305                 310                 315                 320

Leu Asp Met Lys Val Ser Leu Gln Ala Ala Leu Gln Pro Ile Val Ser
                325                 330                 335

Ser Leu Asn Val Ser Val Asp Gly Asn Gly Glu Phe Ile Val Arg Met
            340                 345                 350

Ala Leu Phe Gln Asp Gln Asn Tyr Thr Asn Pro Tyr Glu Gly Asp Ala
        355                 360                 365

Val Glu Leu Ser Val Glu Ser Val Leu Tyr Val Gly Ala Ile Leu Glu
    370                 375                 380

Gln Gly Asp Thr Ser Arg Phe Asn Leu Val Leu Arg Asn Cys Tyr Ala
385                 390                 395                 400

Thr Pro Thr Glu Asp Lys Ala Asp Leu Val Lys Tyr Phe Ile Ile Arg
                405                 410                 415

Asn Ser Cys Ser Asn Gln Arg Asp Ser Thr Ile His Val Glu Glu Asn
            420                 425                 430

Gly Gln Ser Ser Glu Ser Arg Phe Ser Val Gln Met Phe Met Phe Ala
        435                 440                 445

Gly His Tyr Asp Leu Val Phe Leu His Cys Glu Ile His Leu Cys Asp
    450                 455                 460

Ser Leu Asn Glu Gln Cys Gln Pro Ser Cys Ser Arg Ser Gln Val Arg
465                 470                 475                 480

Ser Glu Val Pro Ala Ile Asp Leu Ala Arg Val Leu Asp Leu Gly Pro
                485                 490                 495

Ile Thr Arg Arg Gly Ala Gln Ser Pro Gly Val Met Asn Gly Thr Pro
            500                 505                 510

Ser Thr Ala Gly Phe Leu Val Ala Trp Pro Met Val Leu Leu Thr Val
        515                 520                 525

Leu Leu Ala Trp Leu Phe
    530

<210>  4
<211>  390
<212>  PRT
<213>  Homo sapiens

<220>
<221>  MISC_FEATURE
<223>  Glykoprotein 2

<400>  4

Met Pro His Leu Met Glu Arg Met Val Gly Ser Gly Leu Leu Trp Leu
1               5                   10                  15

Ala Leu Val Ser Cys Ile Leu Thr Gln Ala Ser Ala Val Gln Arg Val
            20                  25                  30

Pro Arg Asp Pro Ser Thr Val Glu Asp Lys Cys Glu Lys Ala Cys Arg
        35                  40                  45

Pro Glu Glu Glu Cys Leu Ala Leu Asn Ser Thr Trp Gly Cys Phe Cys
    50                  55                  60

Arg Gln Asp Leu Asn Ser Ser Asp Val His Ser Leu Gln Pro Gln Leu
65                  70                  75                  80

Asp Cys Gly Pro Arg Glu Ile Lys Val Lys Val Asp Lys Cys Leu Leu
                85                  90                  95

Gly Gly Leu Gly Leu Gly Glu Glu Val Ile Ala Tyr Leu Arg Asp Pro
            100                 105                 110

Asn Cys Ser Ser Ile Leu Gln Thr Glu Glu Arg Asn Trp Val Ser Val
        115                 120                 125

Thr Ser Pro Val Gln Ala Ser Ala Cys Arg Asn Ile Leu Glu Arg Asn
    130                 135                 140

Gln Thr His Ala Ile Tyr Lys Asn Thr Leu Ser Leu Val Asn Asp Phe
145                 150                 155                 160

Ile Ile Arg Asp Thr Ile Leu Asn Ile Asn Phe Gln Cys Ala Tyr Pro
                165                 170                 175

Leu Asp Met Lys Val Ser Leu Gln Ala Ala Leu Gln Pro Ile Val Ser
            180                 185                 190

Ser Leu Asn Val Ser Val Asp Gly Asn Gly Glu Phe Ile Val Arg Met
        195                 200                 205

Ala Leu Phe Gln Asp Gln Asn Tyr Thr Asn Pro Tyr Glu Gly Asp Ala
    210                 215                 220

Val Glu Leu Ser Val Glu Ser Val Leu Tyr Val Gly Ala Ile Leu Glu
225                 230                 235                 240

Gln Gly Asp Thr Ser Arg Phe Asn Leu Val Leu Arg Asn Cys Tyr Ala
                245                 250                 255

Thr Pro Thr Glu Asp Lys Ala Asp Leu Val Lys Tyr Phe Ile Ile Arg
            260                 265                 270

Asn Ser Cys Ser Asn Gln Arg Asp Ser Thr Ile His Val Glu Glu Asn
        275                 280                 285

Gly Gln Ser Ser Glu Ser Arg Phe Ser Val Gln Met Phe Met Phe Ala
    290                 295                 300

Gly His Tyr Asp Leu Val Phe Leu His Cys Glu Ile His Leu Cys Asp
305                 310                 315                 320

Ser Leu Asn Glu Gln Cys Gln Pro Ser Cys Ser Arg Ser Gln Val Arg
                325                 330                 335

Ser Glu Val Pro Ala Ile Asp Leu Ala Arg Val Leu Asp Leu Gly Pro
            340                 345                 350

Ile Thr Arg Arg Gly Ala Gln Ser Pro Gly Val Met Asn Gly Thr Pro
        355                 360                 365

Ser Thr Ala Gly Phe Leu Val Ala Trp Pro Met Val Leu Leu Thr Val
    370                 375                 380

Leu Leu Ala Trp Leu Phe
385                 390

<210>  5
<211>  387
<212>  PRT
<213>  Homo sapiens

<220>
<221>  MISC_FEATURE
<223>  Glykoprotein 2

<400>  5

Met Pro His Leu Met Glu Arg Met Val Gly Ser Gly Leu Leu Trp Leu
1               5                   10                  15

Ala Leu Val Ser Cys Ile Leu Thr Gln Ala Ser Ala Val Gln Arg Asp
            20                  25                  30

Pro Ser Thr Val Glu Asp Lys Cys Glu Lys Ala Cys Arg Pro Glu Glu
        35                  40                  45

Glu Cys Leu Ala Leu Asn Ser Thr Trp Gly Cys Phe Cys Arg Gln Asp
    50                  55                  60

Leu Asn Ser Ser Asp Val His Ser Leu Gln Pro Gln Leu Asp Cys Gly
65                  70                  75                  80

Pro Arg Glu Ile Lys Val Lys Val Asp Lys Cys Leu Leu Gly Gly Leu
                85                  90                  95

Gly Leu Gly Glu Glu Val Ile Ala Tyr Leu Arg Asp Pro Asn Cys Ser
            100                 105                 110

Ser Ile Leu Gln Thr Glu Glu Arg Asn Trp Val Ser Val Thr Ser Pro
        115                 120                 125

Val Gln Ala Ser Ala Cys Arg Asn Ile Leu Glu Arg Asn Gln Thr His
    130                 135                 140

Ala Ile Tyr Lys Asn Thr Leu Ser Leu Val Asn Asp Phe Ile Ile Arg
145                 150                 155                 160

Asp Thr Ile Leu Asn Ile Asn Phe Gln Cys Ala Tyr Pro Leu Asp Met
                165                 170                 175

Lys Val Ser Leu Gln Ala Ala Leu Gln Pro Ile Val Ser Ser Leu Asn
            180                 185                 190

Val Ser Val Asp Gly Asn Gly Glu Phe Ile Val Arg Met Ala Leu Phe
        195                 200                 205

Gln Asp Gln Asn Tyr Thr Asn Pro Tyr Glu Gly Asp Ala Val Glu Leu
    210                 215                 220

Ser Val Glu Ser Val Leu Tyr Val Gly Ala Ile Leu Glu Gln Gly Asp
225                 230                 235                 240

Thr Ser Arg Phe Asn Leu Val Leu Arg Asn Cys Tyr Ala Thr Pro Thr
                245                 250                 255

Glu Asp Lys Ala Asp Leu Val Lys Tyr Phe Ile Ile Arg Asn Ser Cys
            260                 265                 270

Ser Asn Gln Arg Asp Ser Thr Ile His Val Glu Glu Asn Gly Gln Ser
        275                 280                 285

Ser Glu Ser Arg Phe Ser Val Gln Met Phe Met Phe Ala Gly His Tyr
    290                 295                 300

Asp Leu Val Phe Leu His Cys Glu Ile His Leu Cys Asp Ser Leu Asn
305                 310                 315                 320

Glu Gln Cys Gln Pro Ser Cys Ser Arg Ser Gln Val Arg Ser Glu Val
                325                 330                 335

Pro Ala Ile Asp Leu Ala Arg Val Leu Asp Leu Gly Pro Ile Thr Arg
            340                 345                 350

Arg Gly Ala Gln Ser Pro Gly Val Met Asn Gly Thr Pro Ser Thr Ala
        355                 360                 365

Gly Phe Leu Val Ala Trp Pro Met Val Leu Leu Thr Val Leu Leu Ala
    370                 375                 380

Trp Leu Phe
385

## Beschreibung

Die Erfindung betrifft ein Verfahren zum Nachweis von Antikörpern aus Körperflüssigkeiten durch eine lmmunreaktion mit GP2 aus zymogenen Granula des Pankreas, dessen immunreaktive Sequenzen oder Analoga unter Ausschluss von Gewebeschnitten.

Das Verfahren kann zur Diagnose oder Therapiekontrolle von Erkrankungen dienen, die mit einer Immunreaktion gegen diese Substanzen einhergehen. Gegenstand der Erfindung ist daher insbesondere die Verwendung von GP2, dessen immunreaktive Sequenzen oder Analoga zur Diagnose oder Therapiekontrolle von chronisch entzündlichen oder Autoimmunerkrankungen, insbesondere von Morbus Crohn (MC) und Chronischer Pankreatitis (CP).

Die vorliegende Erfindung stützt sich auf die Erkenntnis, dass GP2 ein Autoantigen immuner Prozesse bevorzugt bei MC und CP ist.

MC und Colitis ulcerosa (CU) stellen die zwei wichtigsten entzündlichen Darmerkrankungen (EDE) dar. Sie sind durch chronische, rezidivierende, Gewebe zerstörende Entzündungsprozesse im Verdauungssystem gekennzeichnet. Die Ätiologie und Pathogenese von MC wie auch CU sind bisher unklar.

Während bei der CU die Entzündung vor allem in der Mukosa und Submukosa des Kolon und Rektum auftritt, sind beim MC wanddurchgreifende, granulomatöse Entzündungsprozesse des gesamten Gastrointestinaltrakts charakteristisch. Genetische wie auch Umweltfaktoren scheinen eine entscheidende Rolle bei der Ausbildung von EDE zu besitzen. Der Zusammenhang zwischen Mutationen im NOD2-Gen und Auftreten des MC ist in mehreren Kohorten als gesichert anzusehen. Eine klare Assoziation besteht ebenfalls zum Auftreten des MC im terminalen Ileum. Ein Bezug zwischen genetischen Markern und Therapieverlauf konnte bislang für kein Behandlungsverfahren (inklusive der anti-TNF Therapie) etabliert werden.

Die Inzidenz von MC in Europa liegt bei 5,6 pro 100.000 pro Jahr. Die Prävalenz von MC in Deutschland wird mit 1/500 bis 1/800 angegeben.

Erste Krankheitssymptome von MC treten im Mittel relativ früh mit 30 Jahren auf. MC Patienten sind somit in ihrem Berufsleben betroffen, was entsprechende sozioökonomische Effekte nach sich zieht. Ähnlich wie bei der CU ist bei MC Patienten mit Crohn Colitis und langjährigem Verlauf die Karzinom-Inzidenz erhöht.

Das Beschwerdebild umfasst Unterleibsschmerzen, Durchfall, Malabsorbtion, Abszesse, Fisteln, Gallensteinkomplikationen, Nierensteine und deren Komplikationen.

MC Patienten können eine Reihe von extraintestinalen Manifestationen aufweisen, wobei die Pankreatitis mit 3,5% relativ selten bei MC Patienten vorkommt. Eine Hyperamylasämie und Hyperlipasämie ohne Zeichen einer akuten Pankreatitis kann jedoch bei 8-17% der Patienten beobachtet werden, was auf eine höhere Rate von silenter Pankreatitis hinweist. Vereinzelt sind Veränderungen im Pankreasgang und Einschränkungen der Pankreasfunktion beschrieben worden. Die Höhe der Hyperamylasämie und Hyperlipasämie korreliert mit der Aktivität von MC. Bei 4,6% der MC Patienten findet man gleichzeitig Veränderungen im Gallen- und Pankreasgang ähnlich wie bei Patienten mit Primär Sklerosierender Cholangitis. Die chronische Pankreatitis bei MC Patienten unterscheidet sich allerdings im Allgemeinen von jener bei CU, welche häufiger eine Beteiligung der Gallengänge, Gewichtsverlust und Pankreasgangstenosen aufweist. Es wird die Existenz einer idiopathischen chronischen Pankreatitis, welche mit MC assoziiert ist, diskutiert. Im Unterschied zur CU assoziierten chronischen Pankreatitis treten bei MC Patienten die intestinalen Symptome häufiger vor dem Erscheinen pankreatischer Befunde auf. Die häufige exokrine Pankreasinsuffizienz bei MC lässt sich leicht auf die ausgeprägte azinöse Degeneration zurückführen, die mit dichten Entzündungsinfiltraten im Parenchym einhergeht.

Als Therapie wird die Gabe von 5-Aminosalizylsäure empfohlen, auch wenn in unterschiedlichen Studien nur begrenzte und gelegentlich auch keine Effekte erzielt wurden. Die Anwendung erscheint jedoch bei Patienten mit leichtem bis mäßig schwerem Schub aufgrund der vorhandenen Daten durchaus gerechtfertigt, wenn man bei Wirkungslosigkeit rechtzeitig einen Therapiewechsel einleitet. Bei schwerem Schub ohne Komplikationen ist die Gabe von Prednisolonäquivalenten in Erwägung zu ziehen. Liegen häufige Schübe (≥ 2/Jahr) vor, kann zusätzlich Azathioprin oder 6-Mercaptopurin gegeben werden.

Die Gesamtkosten eines Patienten mit MC werden in Deutschland auf 20.000 EUR pro Jahr und Fall geschätzt. Die Aufwendungen für MC Patienten einschließlich indirekter Kosten belaufen sich in Deutschland auf geschätzte 2 Milliarden EUR, in den USA werden für beide EDE 2,6 Milliarden US Dollar als sozioökonomische Kosten angegeben.

Anti-TNFalpha Präparate sind bei MC wirksam und induzieren die Remission der chronischen Erkrankung. Sie werden jedoch aufgrund der potentiellen Nebenwirkungen als Reservemedikamente in Abhängigkeit von der klinischen Situation empfohlen. MC Patienten mit aktiver Spondylathropathie als extraintestinale Komplikation scheinen von einer anti-TNFalpha Therapie hinsichtlich beider Beschwerdebilder zu profitieren.

Für eine adäquate Therapie und Verlaufskontrolle dieser Patienten ist eine klare Diagnosestellung notwendig. Die klinische Diagnostik des MC beinhaltet als essentiellen Bestandteil eine Ileokoloskopie mit Segmentbiopsien, welche auch vor selektiven Darmoperationen indiziert ist. Sie ist im Verlauf jedoch nicht regelmäßig bei jeder akuten Symptomatik bzw. vor einer neuen antientzündlichen Therapie erforderlich. Eine obere endoskopische Diagnostik sollte in der Primärdiagnostik bei jedem Patienten erfolgen.

Im Rahmen der Diagnostik bilden histologische Untersuchungen von Mukosabiopsien einen wichtigen Baustein. Dafür werde Biopsien vor allem aus makroskopisch auffälligen und unauffälligen Arealen entnommen. Um die Möglichkeiten der histopathologischen Differentialdiagnostik effizient nutzen zu können, werden Biopsien aus mindestens fünf verschiedenen anatomischen Segmenten des gesamten Kolon einschließlich des Rektum, aus dem terminalen Ileum und aus dem oberen Magen-Darm-Trakt empfohlen.

Der transabdominelle Ultraschall als bildgebendes Verfahren dient als sensitives Verfahren zum Nachweis entzündlicher Darmwandveränderungen und der Detektion von Abszessen, Fisteln und Stenosen bei MC Patienten. Ein nachweisbarer erhöhter Blutfluss sowohl in den Mesenterialarterien als auch in der Darmwand ist mit dem Vorliegen akuter Entzündung assoziiert. Endorektaler Ultraschall und die Magnetresonanztomographie (MRT) des kleinen Beckens sind als gleichwertig sensitive Verfahren zur Diagnostik und Klassifikation anorektaler Fisteln und Abszesse anerkannt.

In der Labordiagnostik des MC stellen die Bestimmung von C-reaktivem Protein (CRP), Thrombozyten, Hämoglobin (Hb)/Hämatokrit sowie Leukozyten die Basisdiagnostik dar. Weitere Parameter wie das Differential-Blutbild und das Albumin können eine sinnvolle Ergänzung sein. In der Akutphase des MC sind die oben genannten Parameter wie CRP und die Leukozytenzahl sowie Akutphasen-Proteine bei vielen Patienten erhöht und werden auch für die Verlaufskontrolle empfohlen.

In der Akutphase kommt es zu einem Anstieg der Darmpermeabilität, der alpha1-Antitrypsin-Clearance und der Ausscheidung von Calprotectin im Stuhl.

Die Erhebung klinischer und histologischer Daten erlauben jedoch nicht die klare Unterscheidung von MC und CU und führt häufig zur Definition einer Colitis indeterminata (CI). Weiterhin können Darminfektionen wie auch funktionelle Erkrankungen ähnliche Symptome entwickeln und die Differentialdiagnose erschweren. Bei 10 bis 15% der Patienten mit EDE ist die Einteilung in CD oder MC aufgrund der Biopsiedaten und einer gewissen Überlappung klinischer Symptome im Bereich des Kolon schwierig. Patienten mit Cl scheinen häufiger langfristigere Komplikationen und Anastomoseninsuffizienzen nach chirurgischen Eingriffen aufzuweisen als Patienten mit CU. Die Unterscheidung, ob sich CI Patienten prognostisch in Richtung eines MC oder einer CU entwickeln, hat jedoch einen bedeutenden Einfluss auf Prognose und Krankheitsverlauf sowie die Wahl der medikamentösen Therapie und den Zeitpunkt chirurgischer Eingriffe. Im Krankheitsverlauf lässt sich häufig später auf der Basis weiterer klinischer Daten eine Zuordnung vornehmen. Die Unterscheidung von MC und CU ist zum Beispiel die Grundlage für die Entscheidung, ob bei dem Patienten eine ilioanale Pouchanastomose in Betracht gezogen werden kann. Für MC Patienten mit vorwiegendem Befall des Dickdarms (Crohn Colitis) ist dieser chirurgische Eingriff nur in sehr seltenen Fällen angezeigt, während bei der CU diese Methode häufiger indiziert ist. MC Patienten weisen eine deutlich höhere Rate von Anastomoseninsuffizienzen auf, sodass jede chirurgische Intervention gründlich überdacht werden muss.

Im Rahmen der Differentialdiagnose von EDE sind eine Reihe von Antikörpern beschrieben worden, welche mit körpereigenen und Nahrungsmittelantigenen reagieren. Diese Antikörper scheinen keine pathogenetische Rolle zu spielen und nicht die Krankheitsaktivität abzubilden. Allerdings kann die serologische Antikörperdiagnostik eine entscheidende Hilfe bei der Diagnosestellung liefern und vor allem im Fall der Colitis indeterminata essentiell für die Therapieentscheidung sein.

Es wurden Autoantikörper gegen Zytoskeletproteine bei mittels Biopsie bestätigten MC Patienten beschrieben (Mayet et al., 1990). Unter anderem wurden Autoantikörper gegen Cytokeratin 18, Aktin, Vimentin, Desmin und Tropomyosin gefunden. Obwohl Cytokeratin 18 Autoantikörper eine Korrelation zur Krankheitsaktivität aufwiesen, haben sie sich in der Routinediagnostik von EDE wahrscheinlich aufgrund der geringen Spezifität nicht durchgesetzt.

Für MC Patienten sind Autoantikörper gegen Gewebe von exokrinem Pankreas (PAK) und Antikörper gegen Mannan von Saccharomyces cerevisiae (ASCA) als pathognomonisch identifiziert worden (Stöcker et al., 1987; Main et al., 1988). Autoantikörper gegen humane neutrophile Granulozyten (ANCA) und Becherzellen (BAK) werden bevorzugt bei CU Patienten gefunden.

Die Bestimmung von PAK, ANCA und ASCA wird für die Diagnosestellung bei CI als hilfreich eingeschätzt .

EDE spezifische Autoantikörper gegen Pankreasgewebe, Becherzellen und humane neutrophile Granulozyten werden heute jedoch noch mit Hilfe der Immunfluoreszenztechnik (IFT) aufgrund der nicht bekannten, für die Immunreaktion verantwortlichen Autoantigene bestimmt. So wurden mittels dieser Technik bei 27-39% von MC Patienten Autoantikörper gegen Pankreasantigene gefunden. Über die Hälfte der MC Patienten (68%) mit extraintestinalen Komplikationen können PAK aufweisen.

Für den MC ebenfalls spezifische ASCA werden dagegen verstärkt im Enzymimmunoassay (EIA) detektiert, da diese Methode weniger subjektiv in der Auswertung und automatisierbar ist.
Heute werden die einzelnen Antikörperbestimmungen für die serologische Diagnostik des MC als zu insensitiv angesehen. Die Kombination verschiedener Antikörperspezifitäten kann die diagnostische Sensitivität bzw. Spezifität für die Differentialdiagnose von EDE jedoch außerordentlich verbessern und für Cl eine Prognose zulassen.

Für die Kombination von Parametern ist eine gemeinsame Technologieplattform wie die des EIA äußerst vorteilhaft. Das setzt allerdings die Kenntnis des Autoantigens der PAK voraus, welches von den PAK in den Pankreasgewebeschnitten unterschiedlicher Spezies spezifisch erkannt wird.

Es hat verschiedene Ansätze in der Vergangenheit zur Identifizierung der Autoantigene bei MC gegeben, wobei Pankreasantigene aufgrund der bereits genannten relativen hohen Sensitivität der PAK im Mittelpunkt des Interesses standen. PAK wurden mittels Gewebeschnitten unterschiedlicher Spezies (human, Ratte, Affe) in der IFT nachgewiesen. Das lässt hinsichtlich der Phylogenese auf konservierte Epitope schließen, die von den PAK erkannt werden. Fricke et al. beschrieben einen Proteinkomplex bestehend aus mehreren mit PAK reagierenden Untereinheiten mit einem Molekulargewicht (MW) größer als 800 kDa (Fricke et al., 1999). Die Autoren waren jedoch nicht in der Lage, weder das entsprechende Protein noch die im Immunoblot mit PAK reaktiven Untereinheiten mit den MW 16, 18, 19, 24, 27, 29, 31 und 34 kDa zu sequenzieren und damit zu identifizieren. Es wurde vermutet, dass das von PAK erkannte Protein ein großer Proteinkomplex mit mehreren Untergruppen zu sein scheint. Aufgrund von Inhibitionsexperimenten mit verschiedenen Glykoproteinen wurde eine Reaktivität der PAK mit Kohlenhydratketten der putativen Autoantigene ausgeschlossen.

Seibold et al. beschrieben die Reaktivität von PAK gegen ein aus Pankreassaft gereinigtes makromolekulares Antigen mit einem MW von größer als 10⁶ Da (1000 kDa), welches nach Trypsinbehandlung seine PAK Reaktivität verlor (Seibold et al., 1991). Im ELISA mit verschiedenen Pankreasproteinen wie Amylase, Lipase, Phospholipase A und C, Enterokinase, Carboxypeptidase A und B, Chymotrypsin A und B, Chymotrypsinogen, Elastase, Trypsin, Trypsin Inhibitor, Lactoferrin und Kallekrein konnte keine Reaktivität mit PAK festgestellt werden.

Ausdrücklich wurde auf die Notwendigkeit der ausstehenden Identifizierung des (der) Autoantigens(e) des Pankreas hingewiesen, um den Stellenwert von autoimmunen Prozessen in der Pathogenese des MC aufzuklären und die Diskriminierung von unklaren EDE Fällen durch eine entsprechende Labordiagnostik zu unterstützen (Fricke et al., 1999, Seibold et al., 1991). Bis heute ist es jedoch nicht gelungen, das (die) entsprechende(n) Pankreasantigen(e) zu identifizieren (Bossuyt, 2006).

Dieses Problem wird durch die Charakterisierung von GP2 als Autoantigen für MC als auch die assoziierte chronische Pankreatitis und die Verwendung von GP2 in der Diagnostik von EDE gemäß der Ansprüche gelöst, wobei sich vorteilhafte Ausführungsformen der Erfindung aus den Unteransprüchen ergeben.

Die Erfindung betrifft demgemäß ein Molekül gemäß der Sequenz SEQ ID Nr. 1 für die Verwendung als Arzneimittel.

Ein weiterer Aspekt der Erfindung ist die Verwendung des Moleküls GP2 nach SEQ ID Nr. 1 
10 20 30 40 50 60
MVGSGLLWLA LVSCILTQAS AVQRGYGNPI EASSYGLDLD CGAPGTPEAH VCFDPCQNYT

70 80 90 100 110 120
LLDEPFRSTE NSAGSQGCDK NMSGWYRFVG EGGVRMSETC VQVHRCQTDA PMWLNGTHPA

130 140 150 160 170 180
LGDGITNHTA CAHWSGNCCF WKTEVLVKAC PGGYHVYRLE GTPWCNLRYC TDPSTVEDKC

190 200 210 220 230 240
EKACRPEEEC LALNSTWGCF CRQDLNSSDV HSLQPQLDCG PREIKVKVDK CLLGGLGLGE

250 260 270 280 290 300
EVIAYLRDPN CSSILQTEER NWVSVTSPVQ ASACRNILER NQTHAIYKNT LSLVNDFIIR

310 320 330 340 350 360
DTILNINFQC AYPLDMKVSL QAALQPIVSS LNVSVDGNGE FIVRMALFQD QNYTNPYEGD

370 380 390 400 410 420
AVELSVESVL YVGAILEQGD TSRFNLVLRN CYATPTEDKA DLVKYFIIRN SCSNQRDSTI

430 440 450 460 470 480
HVEENGQSSE SRFSVQMFMF AGHYDLVFLH CEIHLCDSLN EQCQPSCSRS QVRSEVPAID

490 500 510 520
LARVLDLGPI TRRGAQSPGV MNGTPSTAGF LVAWPMVLLT VLLAWLF
 zur Prophylaxe, Diagnose, Therapie oder Nachbehandlung von Autoimmunerkrankungen.

Die Erfindung betrifft demgemäß die überraschende Lehre, dass die erfindungsgemäße Sequenz bzw. die sie kodierende Nukleinsäure in der Therapie eingesetzt werden können, um Autoimmunerkrankungen zu behandeln, insbesondere entzündliche Darmerkrankungen, ganz besonders bevorzugt Morbus Crohn, chronische Pankreatitis und Colitis ulcerosa.

In einer bevorzugten Ausführungsform der Erfindung ist die Autoimmunerkrankung ausgewählt aus der Gruppe der entzündlichen Darmerkrankungen und/oder der autoimmunen Lebererkrankungen.

In einer weiteren bevorzugten Ausführungsform der Erfindung ist die entzündliche Darmerkrankung Morbus Crohn oder eine chronisch Pankreatitis.

Der Morbus Crohn gehört zur Gruppe der chronisch-entzündlichen Darmerkrankungen. Es handelt sich um eine vermutlich autoaggressive, chronischgranulomatöse Entzündung, die im gesamten Magen-Darm-Trakt von der Mundhöhle bis zum After auftreten kann. Bevorzugt befallen sind der untere Dünndarm (*terminales Ileum,* Befall in zirka 40 %) und Grimmdarm, seltener Speiseröhre (*Ösophagus*) und Mund. Charakterisierend für Morbus Crohn ist der diskontinuierliche, segmentale Befall (sog. "*skip lesions*") der Darmschleimhaut, d. h. es können gleichzeitig mehrere Darmabschnitte erkrankt sein, die durch gesunde Abschnitte voneinander getrennt sind. Andere Bezeichnungen für die Krankheit sind Enteritis regionalis Crohn, Ileitis terminalis, Enterocolitis regionalis und sklerosierende chronische Enteritis, bzw. die Abkürzungen MC, CD (*Crohn's Disease*) und übergreifend als IBD (*Inflammatory Bowel Disease*). Morbus Crohn im Sinne der Erfindung ist demgemäß jeder Zustand, der sich makroskopisch durch folgende Veränderungen charakterisieren lässt:
- Gartenschlauchphänomen: Durch Fibrosierung verursachte Segmentstenosen
- Pflastersteinphänomen: Entzündete Schleimhaut wechseln sich mit tiefen Ulzerationen ab, wodurch ein pflastersteinartiges Aussehen entsteht.
- Entzündlicher Konglomerattumor: Verschiedene Darmabschnitte verkleben miteinander.

Histologisch (feingeweblich) erkennt man vor allem eine Häufung von Lymphozyten, (eosinophilen) Granulozyten und Histiozyten in der Biopsie des entzündeten Darmgewebes. Angrenzende Lymphknoten sind meist vergrößert. Häufig bilden sich Granulome, die sich in zwei Typen unterscheiden lassen: *Epitheloidzellgranulome* und *Mikrogranulome* (kleiner und ohne zentrale Nekrose).

Morbus Crohn im Sinne der Erfindung lässt sich aber auch diagnostisch charakterisieren. In diesem Falle ist Morbus Crohn im Sinne der Erfindung ein Zustand, bei dem mindestens eines der folgenden Merkmale detektierbar ist:
- Appendizitis: meist ein sich rasch entwickelnder Schmerz im rechten Unterbauch. Häufig eine Temperaturdifferenz > 1°C zwischen rektaler und axillärer Messung.
- Divertikulitis: tastbare Resistenzen bei meist linksseitigem Unterbauchschmerz.
- Yersiniose: Erregernachweis aus dem Stuhl oder aus dem Biopsiematerial, Anstieg des Antikörpertiters.
- Darmtuberkulose: In Mitteleuropa mittlerweile sehr selten. Die Darmtuberkulose geht häufig mit Beteiligung der Lunge einher. Es finden sich "verkäsende" epitheloidzellige Granulome im Biopsiematerial.
- jede andere invasive infektiöse Colitis (Salmonellenenteritis, pseudomembranöse Colitis etc.)

Pankreatitis ist im Sinne der Erfindung eine Entzündung der Bauchspeicheldrüse (*Pankreas*), die akut oder chronisch verlaufen kann.
In den meisten Fällen entsteht die Pankreatitis durch eine Aktivierung von Pankreas-Enzymen innerhalb des Organs. Da es die Aufgabe dieser Enzyme ist, Eiweiße und Fette zu verdauen, beginnt eine Selbstverdauung des Organs. Diese Selbstverdauung führt zur Entzündung der Bauchspeicheldrüse. In schweren Fällen können Blutungen, ernste Gewebeschäden, Infektionen und Zysten entstehen. Eine entzündete Drüse kann dazu führen, dass Enzyme in den Blutstrom eintreten und so die Lungen, das Herz und die Nieren erreichen, wo weitere Schäden auftreten können. Akute Pankreatitis entsteht, wenn sich die Bauchspeicheldrüse plötzlich entzündet, sich dann aber wieder erholt. Einige Patienten leiden mehrmals an akuter Pankreatitis, können sich aber jedes Mal vollständig erholen. Eine akute Pankreatitis tritt plötzlich auf und kann eine ernste, lebensbedrohliche Krankheit sein, die zahlreiche Komplikationen hervorruft, normalerweise erholen sich Patienten aber von einer akuten Pankreatitis. Die Inzidenz beträgt etwa fünf bis zehn Neuerkrankungen pro 100.000 Einwohner pro Jahr.

Sie kommt in zwei Verlaufsformen vor:
1. *ödematöse Pankreatitis:* blander Verlauf mit Schwellung des Organs und geringen Nekrosen im Fettgewebe der Umgebung
*2. hämorrhagisch - nekrotisierende Pankreatitis:* ausgedehnte Nekrosen und Blutungen im Pankreas und in die Umgebung; durch ihr fulminates Krankheitsbild oft auch als *Pankreasapoplexie* bezeichnet

Die morphologische Beurteilung des Pankreas, insbesondere die Differenzierung zwischen ödematöser und nekrotisierender Pankreatitis gelingt am besten mit der kontrastmittelverstärkten Computertomographie. Zur Einteilung des Schweregrades hat sich der Balthazar-Score bewährt (0 bis 10 Punkte).

Die *akute* Pankreatitis kann mehrere Ursachen haben. Am häufigsten sind Gallensteine, die sich in der Mündung des Gallengangs in den Zwölffingerdarm, die gleichzeitig auch die Mündung des Bauchspeicheldrüsengangs ist, vorübergehend oder länger festklemmen. (ca. 45% der akuten Pankreatitiden). Eine ebenfalls sehr gängige Ursache ist chronischer Alkoholmissbrauch (ca. 35%). Bei etwa 15% der Betroffenen lässt sich kein konkreter Auslöser feststellen, in diesen Fällen spricht man von idiopathischer Genese. Daneben kommen auch seltenere Ursachen vor wie:
- Als Nebenwirkung von Medikamenten (z. B. Asparaginase, Azathioprin, Furosemid, Glukokortikoide, Antibiotika (Tetrazykline, Sulfamethoxazol, Trimethoprim), Antikonvulsiva (Valproat, Carbamazepin), Propofol und andere
- Infektionen z. B. Mumps, Coxsackie-Virus, Hepatitis, HIV, Zytomegalie-Virus
- Erhöhter Blutkalziumwert, z. B. bei Nebenschilddrüsenüberfunktion
- Stark erhöhte Blutfette (Triglyzeride)
- iatrogen nach ERCP
- genetisch: Zystische Fibrose

Eine akute Pankreatitis macht sich anfangs durch Schmerzen im (linken bis gesamten) Oberbauch (Epigastrium), die in den Brustkorb ausstrahlen, bemerkbar, die nach einigen Tagen verschwinden. Die Schmerzen sind oft sehr heftig und zum Teil auch anhaltend. Die Schmerzen können plötzlich und intensiv sein, oder als leichte Schmerzen beginnen und nach der Einnahme von Nahrung (durch die Stimulierung des Pankreas bei der Bildung von Pankreasenzymen zwecks Verdauung der Speise) schlimmer werden. Der Bauch kann geschwollen und sehr empfindlich sein. Charakteristisch bei der körperlichen Untersuchung sind ein druckschmerzhaftes Abdomen und ein sog. *Gummibauch,* der durch Meteorismus und (mäßige) Abwehrspannung bedingt ist. Ebenfalls kann es zu Schmerzen im unteren Bereich der Brustwirbelsäule kommen. Dieser Schmerz ist zunächst ähnlich einem leichten Hexenschuss, entwickelt sich aber in der Folge mehr zu einem "Durchstochenwerden", vom Rücken her hin zum Bereich des Pankreaskopfes auf der Bauchseite.
Patienten mit einer akuten Pankreatitis sehen gewöhnlich sehr krank aus und fühlen sich auch so. Andere Symptome sind zum Beispiel Übelkeit, Erbrechen, Obstipation, Fieber, erhöhter Puls. In schweren Fällen treten Gelbsucht (lkterus) (bei Verlegung der Gallenwege), Bauchwassersucht (Aszites) (bei Verlegung des Pfortadersystems), Pleuraergüsse sowie Schock- und Sepsiszeichen hinzu. Labordiagnostisch kann eine erhöhte Leukozytenzahl (Leukozytose) sowie ein Anstieg der Konzentration von Pankreasenzymen (z. B. Trypsin, Amylasen, Lipase) nachgewiesen werden. Auch die Calcium-, Magnesium-, Natrium-, Kalium-, Bikarbonat-, Zucker- oder Fettwerte im Blut können erhöht sein.
Ungefähr 20% der akuten Pankreatitis-Fälle sind ernst. Der Patient kann dehydrieren und einen niedrigen Blutdruck entwickeln. Manchmal kommt es zu Herz-, Lungen- oder Nierenversagen. In den schlimmsten Fällen führt eine akute Pankreatitis zu Blutungen, Schock und manchmal zum Tod.

Nach der aktuellen Atlanta-Klassifikation wird die milde von der schweren akuten Pankreatitis unterschieden.
Eine veraltete Einteilung unterteilte eine ödematöse Form (Frühstadium), eine hämorrhagische Form mit lokalen oder generalisierten Blutungen und eine akute nekrotisierende Form.

Eine chronische Pankreatitis hat viele Ursachen, aber 70 bis 80% sind auf chronischen Alkoholmissbrauch zurückzuführen. Sie tritt häufiger bei Männern als bei Frauen auf und entwickelt sich häufig zwischen dem 30. und dem 40. Lebensjahr. Eine chronische Pankreatitis kann sich auch aus einer akuten Entzündung entwickeln, wenn die Ursache nicht beseitigt wird oder der Ausführungsgang beschädigt ist.
Einige chronische Pankreatitiden sind erblich bedingt. Diese beruhen auf einer Abnormalität der vom Pankreas gebildeten Enzyme, welche das Gewebe schädigen. Andere Formen der Erkrankung haben ihre Ursachen in äußeren Faktoren wie z. B. dem Tabakrauchen.

In den frühen Stadien einer Pankreatitis kann der Arzt häufig nicht entscheiden, ob es sich um eine akute oder eine chronische Form handelt. Die Symptome können die gleichen sein.
Eine chronische Pankreatitis verursacht häufig chronische Schmerzen. In einigen Fällen chronischer Pankreatitis lässt der Schmerz nach, wenn die Krankheit fortschreitet. Sie führt auch zu einer Unterfunktion der Bauchspeicheldrüsen-Aktivität, was zu Gewichtsverlust und Verdauungsstörungen führt. Die ungenügende Verdauung und Resorption führt zur Abgabe von Fett und Eiweiß über den Stuhl. Wenn die endokrinen Zellen (Langerhans-Inseln) im Pankreas geschädigt sind, kann sich ein Diabetes entwickeln.

Eine Diagnose der chronischen Pankreatitis ist schwierig, jedoch stehen einige hochentwickelte medizinische Techniken zu Verfügung. Pankreas-Funktions-Bluttests können helfen, zu entscheiden, ob die Bauchspeicheldrüse noch in der Lage ist, genug Verdauungsenzyme herzustellen. Sie haben sich in der Praxis allerdings kaum durchgesetzt.

Abnormalitäten des Pankreas können auch durch Sonografie, ERCP und Computertomographie erkannt werden.

In fortgeschritteneren Stadien einer chronischen Pankreatitis, wenn Diabetes und fehlerhafte Resorption auftreten, kann der Arzt auch Blut, Urin und Stuhltests durchführen, um eine Diagnose zu stellen.

Eine chronische Pankreatitis wird durch das Verschreiben von Schmerzmitteln und eine Nahrungsumstellung behandelt. Patienten können den Verlust von Fett und Eiweiß reduzieren, indem sie Medikamente einnehmen, die Pankreas-Enzyme enthalten. Dies wird eine verbesserte Ernährung und eine Gewichtszunahme zur Folge haben. Manchmal werden Insulin oder andere Medikamente verschrieben, um den Blutzuckerspiegel zu kontrollieren.

In einigen Fällen von chronischer Pankreatitis wird ein chirurgischer Eingriff vorgenommen, um die Schmerzen zu lindern, indem ein vergrößerter und gestauter Pankreasgang entlastet wird.

In einer weiteren bevorzugten Ausführungsform der Erfindung ist die Lebererkrankung Primär Sklerosierende Cholangitis oder Autoimmunenteritiden.

Cholangitis, auch Cholangiitis im Sinne der Erfindung, bezeichnet eine Entzündung der intrahepatischen Gallengänge. Diese kann durch verschiedene Ursachen ausgelöst werden, unter anderem durch Verstopfungen der Gallenwege durch Gallensteine, Stenosen, Tumore oder Parasitenbefall. Man unterscheidet dabei die akute eitrige Cholangitis, die nichteitrige destruierende Cholangitis sowie die chronisch sklerosierende Cholangitis.

Akute eitrige Cholangitis: Die akute Cholangitis entsteht meist durch eine Infektion bei einer Besiedlung durch Bakterien, meist *Escherichia coli, Enterococcus-* oder Klebsiella-Arten. Als Symptome treten ein einseitiger Schmerz des Oberbauches, Fieber und Schüttelfrost auf. Bei einer schweren eitrigen Cholangitis kommt es außerdem zu Schockzuständen, Störungen des Zentralnervensystems sowie Nierenfunktionsstörungen. Die Behandlung beinhaltet endoskopische Interventionen an den Gallenwegen wie die endoskopisch-retrograde Cholangiopankreatikographie (ERCP) oder perkutane transhepatische Cholangiodrainage (PTCD), die den Gallefluss wieder herstellen, und in aller Regel eine Antibiose.

Nicht eitrige destruierende Cholangitis: Diese Form der Cholangitis verläuft chronisch und wird auch als primär biliäre Zirrhose bezeichnet. 95% der Betroffenen sind Frauen, wobei der Häufigkeitsgipfel zwischen dem 40. und 60. Lebensjahr liegt. Diagnostisch findet man bei den meisten Patienten antimitochondriale Antikörper (AMA) im Blut, weswegen eine autoimmunologische Genese angenommen wird. Die Patienten sind klinisch durch Juckreiz, Ikterus und Hypercholesterinämie gekennzeichnet. Im späteren Verlauf kann den Patienten nur noch mittels Lebertransplantation geholfen werden.

Chronisch sklerosierende Cholangitis: Die chronisch sklerosierende Cholangitis ist die seltenste Gallenwegsentzündung und wird in eine primär und eine sekundär sklerosierende Form eingeteilt. Die primäre Form entsteht durch Infekte bei bereits bestehender genetischer Disposition (es wurde eine Assoziation mit dem Antigen HLA-B8 festgestellt) und betrifft Männer doppelt so häufig wie Frauen. In bis 90% der Fälle findet man p-ANCA. Die sekundäre Form entsteht auf dem Boden bereits bestehender Immundefizienzsyndrome. Wie bei der destruierenden Cholangitis ist auch hier im Endstadium eine Lebertransplantation nötig.

Autoimmunenteritiden im Sinne der Erfindung ist jede Form der Enteritis, insbesondere solche, die im wesentlichen durch chronisch-entzündliche Darmerkrankungen hervorgerufen werden. Im Sinne der Erfindung sind Autoimmunenteritiden aber auch solche, die durch Salmonellen, E. Coli, Cholera- oder Typhuserreger hervorgerufen werden, oder aber durch Pilze, Protozoen, toxische Substanzen, aber auch jede allergisch bedingte Enteritis oder jede Form der aktinischen Enteritis, der Yersinia-Enteritis oder der bakteriellen Ruhr.

In einer weiteren bevorzugten Ausführungsform der Erfindung ist die Aminosäuresequenz mindestens zu 60%, vorzugsweise 70%, bevorzugt 80%, ganz besonders bevorzugt 90% homolog zu der Sequenz gemäß SEQ ID Nr. 1. D. h., die Erfindung betrifft sämtliche Peptide, die bevorzugt 60%, 70%, 80%, ganz besonders bevorzugt 90% Homologie zu der Sequenz gemäß SEQ ID Nr. 1 aufweisen. Selbstverständlich können diese Homologen durch Deletion, Addition, Substitution, Translokation, Inversion und/oder Insertion modifiziert sein. Diese Modifikation betrifft insbesondere homologe Peptide, die funktionsanalog sind. Funktionsanalog sind die Peptide im Sinne der Erfindung, wenn sie mit Autoantikörpern, die mit den o. g. Krankheiten assoziiert sind, spezifisch interagieren.

Erfindungsgegenstand sind demgemäß das offenbarte Peptid sowie die Homologen, die funktionsanalog eingesetzt werden können, sowie ihre Verwendung; d. h. auch die Offenbarung von zweckgebundenen Stoffen für die erste medizinische Indikation, wie auch die Verwendung in der Forschung und für weitere medizinische Anwendungen. Dem Fachmann ist in Bezug auf Homologe/Funktionsanaloge bekannt, dass er Änderungen durch Additionen, Deletionen oder Substitutionen vornehmen kann, ohne dass das Polypeptid im wesentlichen verändert wird. Nicht wesentlich verändert ist die modifizierte Aminosäuresequenz, wenn sie die gleiche Funktion erfüllt, wie die Sequenz gemäß SEQ ID Nr. 1 und zwar im wesentlichen auf dieselbe Art und Weise, wobei sie hierbei zum selben Ergebnis führt. D. h., eine modifizierte Aminosäuresequenz im Sinne der Erfindung ist jede veränderte Sequenz, wobei ihre Anwendung keine wesentliche Auswirkung auf die Lösung des erfindungsgemäßen Problems hat und wenn dies für den Fachmann offensichtlich ist, wobei der Fachmann hierbei erkennt, dass die Erfinder nicht die Absicht haben, ihre Lehre auf den Wortlaut der Ansprüche - d. h. auf die Sequenz gemäß SEQ ID Nr.1 - zu beschränken.

Funktionsanaloge Peptide können beispielsweise SEQ ID Nr. 2 
MPHLMERMVGSGLLWLALVSCILTQASAVQRGYGNPIEASSYGLDLDCGAPGTPEAHVCF
DPCQNYTLLDEPFRSTENSAGSQGCDKNMSGWYRFVGEGGVRMSETCVQVHRCQTDAP
MWLNGTHPALGDGITNHTACAHWSGNCCFWKTEVLVKACPGGYHVYRLEGTPWCNLRYC
TVPRDPSTVEDKCEKACRPEEECLALNSTWGCFCRQDLNSSDVHSLQPQLDCGPREIKVK
VDKCLLGGLGLGEEVIAYLRDPNCSSILQTEERNWVSVTSPVQASACRNILERNQTHAIYKN
TLSLVNDFIIRDTILNINFQCAYPLDMKVSLQAALQPIVSSLNVSVDGNGEFIVRMALFQDQNY
TNPYEGDAVELSVESVLYVGAILEQGDTSRFNLVLRNCYATPTEDKADLVKYFIIRNSCSNQ
RDSTIHVEENGQSSESRFSVQMFMFAGHYDLVFLHCEIHLCDSLNEQCQPSCSRSQVRSE
VPAIDLARVLDLGPITRRGAQSPGVMNGTPSTAGFLVAWPMVLLTVLLAWLF^
 oder SEQ ID Nr. 3 
MPHLMERMVGSGLLWLALVSCILTQASAVQRGYGNPIEASSYGLDLDCGAPGTPEAHVCF
DPCQNYTLLDEPFRSTENSAGSQGCDKNMSGWYRFVGEGGVRMSETCVQVHRCQTDAP
MWLNGTHPALGDGITNHTACAHWSGNCCFWKTEVLVKACPGGYHVYRLEGTPWCNLRYC
TDPSTVEDKCEKACRPEEECLALNSTWGCFCRQDLNSSDVHSLQPQLDCGPREIKVKVDK
CLLGGLGLGEEVIAYLRDPNCSSILQTEERNWVSVTSPVQASACRNILERNQTHAIYKNTLSL
VNDFIIRDTILNINFQCAYPLDMKVSLQAALQPIVSSLNVSVDGNGEFIVRMALFQDQNYTNP
YEGDAVELSVESVLYVGAILEQGDTSRFNLVLRNCYATPTEDKADLVKYFIIRNSCSNQRDS
TIHVEENGQSSESRFSVQMFMFAGHYDLVFLHCEIHLCDSLNEQCQPSCSRSQVRSEVPAI
DLARVLDLGPITRRGAQSPGVMNGTPSTAGFLVAWPMVLLTVLLAWLF
 oder SEQ ID Nr. 4 
MPHLMERMVGSGLLWLALVSCILTQASAVQRVPRDPSTVEDKCEKACRPEEECLALNSTW
GCFCRQDLNSSDVHSLQPQLDCGPREIKVKVDKCLLGGLGLGEEVIAYLRDPNCSSILQTEE
RNWVSVTSPVQASACRNILERNQTHAIYKNTLSLVNDFIIRDTILNINFQCAYPLDMKVSLQA
ALQPIVSSLNVSVDGNGEFIVRMALFQDQNYTNPYEGDAVELSVESVLYVGAILEQGDTSRF
NLVLRNCYATPTEDKADLVKYFIIRNSCSNQRDSTIHVEENGQSSESRFSVQMFMFAGHYD
 
LVFLHCEIHLCDSLNEQCQPSCSRSQVRSEVPAIDLARVLDLGPITRRGAQSPGVMNGTPS
TAGFLVAWPMVLLTVLLAWLF
 oder aber SEQ ID Nr. 5 
MPHLMERMVGSGLLWLALVSCILTQASAVQRDPSTVEDKCEKACRPEEECLALNSTWGCF
CRQDLNSSDVHSLQPQLDCGPREIKVKVDKCLLGGLGLGEEVIAYLRDPNCSSILQTEERN
WVSVTSPVQASACRNILERNQTHAIYKNTLSLVNDFIIRDTILNINFQCAYPLDMKVSLQAALQ
PIVSSLNVSVDGNGEFIVRMALFQDQNYTNPYEGDAVELSVESVLYVGAILEQGDTSRFNLV
LRNCYATPTEDKADLVKYFIIRNSCSNQRDSTIHVEENGQSSESRFSVQMFMFAGHYDLVFL
HCEIHLCDSLNEQCQPSCSRSQVRSEVPAIDLARVLDLGPITRRGAQSPGVMNGTPSTAGF
LVAWPMVLLTVLLAWLF
 sein. Die genannten Peptide werden für die Verwendung in der Forschung, aber auch für medizinische Anwendungen, insbesondere für die oben ausgeführten Immunerkrankungen beansprucht. Die genannten Sequenzen erfüllen im Wesentlichen dieselbe Funktion auf im Wesentlichen demselben Weg und bringen im Wesentlichen dasselbe Ergebnis hervor wie die Sequenz SEQ ID Nr. 1. Sie werden daher von der erfindungsgemäßen Lehre, der Verwendung des Moleküls GP2 für die Verwendung als Arzneimittel, insbesondere zur Prophylaxe, Diagnose, Therapie und/oder Nachbehandlung von Immunerkrankungen, erfasst.

Die Aminosäuresequenzen, d. h. die Peptide die im Sinne der Erfindung, können daher soviel weiter Aminosäuren, Spacer oder andere Strukturen umfassen, dass sie geeignet sind, mit den Antikörpern, bevorzugt Autoantikörpern, zu interagieren, vorzugsweise so, dass sie ein Epitop für diese darstellen. Die erfindungsgemäße Sequenz ist demgemäß nicht auf die Peptide beschränkt, die Antikörper-Epitope betreffen, sondern sie bezieht sich auf das Molekül und alle Bruchstücke hiervon, die mit Autoantikörpern spezifisch wechselwirken. Die Begriffe Epitop und Peptid sowie Aminosäuresequenz können daher in bevorzugten Ausführungsformen im Sinne der Erfindung synonym verwendet werden.

Im Stand der Technik sind verschiedene Möglichkeiten zur Herstellung von funktionsanalogen Peptiden offenbart. Peptide, die von den erfindungsgemäßen Peptiden ausgehend mit solchen Verfahren designt werden, sind von der erfindungsgemäßen Lehre mit erfasst. Eine Möglichkeit des Generierens von funktionsanalogen Peptiden ist beispielsweise in PNAS USA 1998, Oct. 13; 9521:12179-84, WO 99/6293 und/oder WO 02/38592 beschrieben; diese Lehren sind in den Offenbarungsgehalten der Erfindung mit aufgenommen. Das heißt, sämtliche Peptide, Peptidfragmente oder Strukturen, die Peptide umfassen, die mit den genannten Verfahren - von den erfindungsgemäßen Peptiden ausgehend - generiert wurden, sind Peptide im Sinne der Erfindung, sofern sie die erfindungsgemäße Aufgabe lösen, insbesondere mit den krankheitsverursachenden Autoantikörpern wechselwirken. Bei diesen Autoantikörpern kann es sich beispielsweise um agonistische Autoantikörper handeln, die Rezeptoren aktivieren.

In einer weiteren bevorzugten Ausführungsform der Erfindung umfasst das Molekül einen Linker oder Spacer, ausgewählt aus der Gruppe α-Aminocarbonsäuren sowie deren Homo- und Heterooligomere; α,ω-Aminocarbon-säuren sowie deren verzweigte Homo- oder Hetero-oligomere; sonstige Aminosäuren sowie die linearen und verzweigten Homo- oder Heterooligomere; Amino-oligoalkoxy-alkylamine; Maleinimidocarbonsäure-Derivate; Oligomere von Alkylaminen; 4-Alkylphenyl-Derivate; 4-Oligoalkoxyphenyl- oder 4-Oligoalkoxy-phenoxy-Derivate; 4-Oligoalkylmercaptophenyl- oder 4-Oligoalkylmercaptophenoxy-Derivate; 4-Oligoalkyl-aminphenyl- oder 4-Oligoalkylaminyphenoxy-Derivate; (Oligoalkylbenzyl)-phenyl- oder 4-(Oligo-alkylbenzyl)-phenoxy-Derivate sowie 4-(Oligoalkoxy-benzyl)-phenyl- oder 4-(Oligoalkoxybenzyl)-phenoxy-Derivate; Trityl-Derivate; Benzyloxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-Phenoxylalkyl- oder Oligoalkoxy-phonxyalkyl-Derivate; Carbamat-Derivate; Amine; Trialkylsilyl- oder Dialkyl-akoxysilyl-Derivate; Alkyl- oder Aryl-Derivate oder Kombinationen davon.

In einer weiteren bevorzugten Ausführungsform der Erfindung wird das Molekül GP2 zur Herstellung eines Arzneimittels zur Behandlung von Autoimmunerkrankungen eingesetzt.

In einer weiteren bevorzugten Ausführungsform der Erfindung kann das Molekül GP2 löslich oder festphasengebunden zum direkten oder indirekten Autoantikörpernachweis in Körperflüssigkeiten, insbesondere Blut oder Serum, verwendet werden.

In einer weiteren bevorzugten Ausführungsform der Erfindung werden zusätzlich zu dem löslichen oder festphasengebundenen Molekül GP2 unspezifische Adsorbermoleküle verwendet, ausgewählt aus der Gruppe umfassend Protein A, Protein G, Anti-Human-Immunglobuline oder L-Tryptophan.

Bevorzugt wird die anmeldungsgemäße Sequenz bzw. Bruchstücke hieraus als therapeutischer Wirkstoff eingesetzt. Die Verwendung als therapeutischer Wirkstoff im Sinne der Erfindung meint die Anwendung der Aminosäuresequenz bzw. der Peptide, die aus dieser gebildet werden können, auf dem gesamten Gebiet der Medizin.

In einer weiteren bevorzugten Ausführungsform der Erfindung sind insbesondere die anmeldungsgemäße Sequenz bzw. Peptide, die aus dieser generiert werden können, immobilisiert. Im Sinne der Erfindung werden unter Immobilisierung verschiedene Verfahren und Techniken zum Fixieren der Peptide auf bestimmten Trägern beispielsweise gemäß der WO 99/56126 oder der WO 02/26292 verstanden. Die Immobilisierung kann beispielsweise der Stabilisierung der Peptide dienen, wodurch diese insbesondere bei Lagerung oder bei einmaligem Batch-Ansatz durch biologische, chemische oder physikalische Einwirkungen in ihrer Aktivität nicht reduziert oder nachteilig modifiziert werden. Durch die Immobilisierung der Peptide ist ein wiederholter Einsatz unter technischen oder klinischen Routine-Bedingungen möglich; weiterhin kann eine Probe - bevorzugt Blutbestandteile - mit mindestens einem der erfindungsgemäßen Peptide kontinuierlich umgesetzt werden. Dies kann insbesondere durch verschiedene Immobilisierungstechniken erreicht werden, wobei die Bindung der Peptide an andere Peptide oder Moleküle bzw. an einen Träger so erfolgt, dass die dreidimensionale Struktur, insbesondere an dem Zentrum, das die Wechselwirkung mit den Autoantikörpern vermittelt, der entsprechenden Moleküle, insbesondere der Peptide, nicht verändert wird. Vorteilhafterweise geht die Spezifität zu den Autoantikörpern der Patienten durch die Immobilisierung nicht verloren. Im Sinne der Erfindung können drei grundsätzliche Methoden zur Immobilisierung verwendet werden:
(i) Quervernetzung: Bei der Quervernetzung werden die Peptide miteinander fixiert, ohne dass ihre Aktivität nachteilig beeinflusst wird. Sie sind vorteilhafterweise durch die Quervernetzung nicht mehr löslich.
(ii) Bindung an einen Träger: Die Bindung an einen Träger erfolgt zum Beispiel durch Adsorption, lonenbindung oder kovalente Bindung. Dies kann auch innerhalb von mikrobiellen Zellen bzw. Liposomen oder anderen membranhaltigen geschlossenen bzw. offenen Strukturen erfolgen. Die Peptide werden durch die Fixierung vorteilhafterweise nicht in ihrer Aktivität beeinflusst. Die Peptide können mit Vorteil zum Beispiel in der Klinik in Diagnose oder Therapie trägergebunden mehrfach oder kontinuierlich eingesetzt werden.
(iii) Einschluss: Der Einschluss erfolgt im Sinne der Erfindung insbesondere an eine semipermeable Membran in Form von Gelen, Fibrillen oder Fasern. Gekapselte Peptide sind durch eine semipermeable Membran so durch die umgebende Probenlösung getrennt, dass sie vorteilhafterweise noch mit den Autoantikörpern oder mit Fragmenten dieser interagieren können. Für die Immobilisierung stehen verschiedene Verfahren zur Verfügung, wie beispielsweise die Adsorption an einen inerten oder elektrisch geladenen anorganischen oder organischen Träger. Solche Träger können beispielsweise poröse Gele, Aluminiumoxid, Betonid, Agarose, Stärke, Nylon oder Polyacrylamid sein. Die Immobilisierung erfolgt hierbei durch physikalische Bindungskräfte, oft unter Beteiligung von hydrophoben Wechselwirkungen und ionischen Bindungen. Derartige Methoden sind vorteilhafterweise einfach zu handhaben und sie beeinflussen die Konformation der Peptide nur in geringem Umfang. Durch elektrostatische Bindungskräfte zwischen den geladenen Gruppen der Peptide und dem Träger kann die Bindung vorteilhafterweise verbessert werden, zum Beispiel durch die Verwendung von lonenaustauschern, insbesondere Sephadex.

Ein weiteres Verfahren ist die kovalente Bindung an Trägermaterialien. Die Träger können dazu reaktive Gruppen aufweisen, die mit Aminosäure-Seitenketten homöopolare Bindungen eingehen. Geeignete Gruppen in Peptiden sind Carboxy-, Hydroxy- und Sulfidgruppen und insbesondere die endständigen Aminogruppen von Lysinen. Aromatische Gruppen bieten die Möglichkeit für Diazo-Kopplungen. Die Oberfläche von mikroskopischen porösen Glaspartikeln kann durch Behandlung mit Silanen aktiviert und anschließend mit Peptiden umgesetzt werden. HydroxyGruppen natürlicher Polymere können zum Beispiel mit Bromzyan aktiviert und anschließend mit Peptiden gekoppelt werden. Mit Polyacrylamid-Harzen können zahlreiche Peptide vorteilhafterweise direkte kovalente Bindungen eingehen. Bei dem Einschluss in dreidimensionale Netzwerke werden die Peptide in ionotrophe Gele oder andere dem Fachmann bekannte Strukturen eingeschlossen. Die Poren der Matrix sind insbesondere so beschaffen, dass die Peptide zurückgehalten werden und eine Interaktion mit den Ziel-Molekülen möglich ist. Bei der Quervernetzung werden die Peptide durch Vernetzung mit bifunktionellen Agenzien in polymere Aggregate umgewandelt. Derartige Strukturen sind gelatinös und leicht verformbar und insbesondere für den Einsatz in verschiedenen Reaktoren geeignet. Durch Zugabe anderer inaktiver Komponenten, wie zum Beispiel Gelatine, bei der Vernetzung können die mechanischen und Bindungseigenschaften vorteilhafterweise verbessert werden. Bei der Mikroverkapselung wird der Reaktionsraum der Peptide mit Hilfe von Membranen eingegrenzt. Die Mikroverkapselung kann zum Beispiel als Grenzflächen-Polymerisation durchgeführt werden. Durch die Immobilisierung bei der Mikroverkapselung werden die Peptide unlöslich und dadurch wieder verwendbar. Im Sinne der Erfindung sind immobilisierte Peptide alle Peptide, die sich in einem Zustand befinden, der ihre Wiederverwendung erlaubt. Die Einschränkung der Beweglichkeit und der Löslichkeit der Peptide auf chemischem, biologischem oder physikalischem Wege führt vorteilhafterweise zu niedrigen Verfahrenskosten, insbesondere bei der Eliminierung von Autoantikörpern aus Blutbestandteilen.

In einer weiteren bevorzugten Ausführungsform der Erfindung ist das Peptid bzw. die gesamte Aminosäuresequenz an eine Festphase gebunden. Die Bindung des Peptides bzw. der gesamten Aminosäuresequenz an die Festphase kann über einen Spacer erfolgen. Als Spacer können alle chemischen Verbindungen eingesetzt werden, die für die Funktion des Spacers die geeigneten strukturellen und funktionellen Voraussetzungen aufweisen, solange sie nicht das Bindeverhalten derart modifizieren, dass eine Bindung des Autoantikörpers mit dem Peptid nachteilhafterweise beeinträchtigt wird.

Die pharmazeutische Zusammensetzung kann insbesondere als Arzneimittel eingesetzt werden. Hierzu ist es beispielsweise möglich, die Peptide oder die komplette Aminosäuresequenz durch Zyklisierung oder andere dem Fachmann bekannte Verfahren so zu modifizieren, dass sie durch körpereigene peptidabbauende Strukturen, wie zum Beispiel Serumproteasen, nicht zerstört werden können. Durch Verwendung der erfindungsgemäßen Peptide oder des Proteins (SEQ ID Nr. 1) ist es möglich, die Autoantikörper in oder ex vivo zu neutralisieren. Bei einer in vivo Neutralisation werden die Arzneimittel dem Patienten direkt verabreicht, bei einer ex vivo Neutralisation wird beispielsweise das Blut über eine Schleife - zum Beispiel in Form eines Schlauch-Kreislaufes - aus dem Körper geleitet, folgend mit dem Arzneimittel in Kontakt gebracht und nach der erfolgten Neutralisation der Autoantikörper wieder in den Organismus, insbesondere dem Patienten, zurückgeführt. Im Sinne der Erfindung gelten als Arzneimittel sowohl solche pharmazeutischen Zusammensetzungen, die für die therapeutischen und prophylaktischen Zwecke verwendet werden als auch solche pharmazeutischen Zusammensetzungen, die als Diagnostikum eingesetzt werden können.

Arzneimittel oder pharmazeutische Zusammensetzungen, die vorliegend synonym verwendet werden, sind erfindungsgemäß Stoffe und Zubereitungen aus Stoffen, die dazu bestimmt sind, durch Anwendung am oder im menschlichen Körper Krankheiten, Leiden, Körperschäden oder krankhafte Beschwerden zu heilen, zu lindern oder zu verhüten. Medizinische Hilfsstoffe sind erfindungsgemäß solche Stoffe, die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Beispiele für pharmazeutisch verträgliche Träger sind nachstehend aufgeführt. Die Arzneimittelformulierung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen zum Beispiel Phosphat-gepufferte Kochsalzlösungen, Wasser, Emulsionen wie zum Beispiel Öl/Wasser-Emulsionen, verschiedene Arten von Detergenzien, sterile Lösungen, etc. Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, beispielsweise in einem Bereich von 1 µg bis 10 g an Peptiden oder dem Protein pro Tag und Patient. Bevorzugt werden dabei Dosen von 1 mg bis 1 g. Bevorzugt wird eine Verabreichung von möglichst wenigen und niedrigen Dosen und weiter bevorzugt eine einmalige Dosis. Die Verabreichung kann auf verschiedenen Wegen erfolgen, beispielsweise intravenös, intraperitoneal, intrarektal, intragastrointestinal, intranodal, intramuskulär, lokal, aber auch subkutan, intradermal oder auf der Haut oder über die Schleimhäute. Die Verabreichung von Nukleinsäuren, die für das erfindungsgemäße Peptid codieren, kann auch in Form von Gen-Therapie geschehen, beispielsweise über virale Vektoren. Die Art der Dosierung und des Verabreichungsweges kann vom behandelnden Arzt entsprechend den klinischen Faktoren bestimmt werden. Es ist dem Fachmann bekannt, dass die Art der Dosierung von verschiedenen Faktoren abhängig ist, wie zum Beispiel der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden. Weiterhin ist dem Fachmann bekannt, dass er die Konzentration der Autoantikörper mit den erfindungsgemäßen Peptiden zunächst diagnostizieren kann, um die notwendige Konzentration des Arzneimittels zu bestimmen.

Die pharmazeutischen Zusammensetzungen oder das Arzneimittel umfassen insbesondere eine pharmakologische Substanz, die ein oder mehrere erfindungsgemäße Peptide oder dem Protein oder/und diese kodierenden Nukleinsäuremoleküle in einer geeigneten Lösung oder Verabreichungsform enthält. Diese können entweder alleine mit den entsprechenden unter Arzneimitteln oder pharmazeutischen Zusammensetzungen beschriebenen Hilfsstoffen oder in Kombination mit einem oder mehreren Adjuvantien, beispielsweise QS-21, GPI-0100 oder andere Saponine, Wasser-Öl Emulsionen wie beispielsweise Montanide, Adjuvantien, Polylysin, Polyargininverbindungen, DNA-Verbindungen wie beispielsweise CpG,Detox, bakterielle Vakzine wie beispielsweise Thyphusvakzine oder BCG-Vakzine, Salze wie beispielsweise Kalziumphosphate und/oder einem anderen geeigneten Stoff zur Wirkungsverstärkung verabreicht werden ; vorzugsweise immunstimulatorische Moleküle, wie Interleukine, beispielsweise IL-2, IL-12, IL-4 und/oder Wachstumsfaktoren, beispiels- weise GM-CSF. Diese werden in bekannten Methoden mit den erfindungsgemäßen Peptiden oder Erkennungsmolekülen gemischt und in einer geeigneten Formulierung und Dosierung verabreicht. Formulierungen, Dosierungen und geeignete Komponenten sind dem Fachmann bekannt.

Die pharmazeutische Zusammensetzung oder das Arzneimittel kann selbstverständlich auch eine Kombination von zwei oder mehreren der erfindungsgemäßen pharmazeutischen Zusammensetzungen oder Arzneimittel sein, sowie eine Kombination mit anderen Arzneimitteln, wie beispielsweise Antikörpertherapien, Chemotherapien oder Radiotherapien, die auf eine geeignete Weise zeitlich gemeinsam oder getrennt verabreicht bzw. angewandt werden. Die Herstellung der Arzneimittel oder pharmazeutischen Zusammensetzungen erfolgt nach an sich bekannten Methoden.

Die Erfindung betrifft auch einen Kit umfassend das Nukleinsäuremolekül, den Vektor, das Peptid und/oder das Protein, gegebenenfalls mit einer Anleitung oder Information zur pharmazeutischen Bereitstellung bzw. zum therapeutischen Behandlungsverfahren. Die Information kann beispielsweise ein Beipackzettel sein oder ein anderes Medium, was dem Anwender Informationen darüber gibt, in welchem therapeutischen Verfahren die genannten Substanzen einzusetzen sind. Der Beipackzettel enthält insbesondere detaillierte und/oder wesentliche Informationen über das Heilverfahren. Selbstverständlich ist es nicht zwingend erforderlich, dass die Information einen Beipackzettel darstellt, es ist möglich, dass diese Information beispielsweise über das Internet mitgeteilt wird.

Die Erfindung betrifft auch eine Vorrichtung zur Chromatographie, die die erfindungsgemäßen Peptide umfasst.

In einer bevorzugten Ausführungsform sind die Peptide innerhalb des Chromatographiesystems an eine Festphase gebunden.

Die erfindungsgemäße Vorrichtung kann insbesondere dazu verwendet werden, die Autoantikörper aus Flüssigkeiten eines Patienten zu eliminieren bzw. die Autoantikörper zu neutralisieren. Dieses Verfahren ist dem Fachmann unter dem Begriff der Immunadsorption und der Apheresetherapie bekannt. Mit Hilfe der Immunadsorption werden Immunglobuline aus dem Blut des Patienten entfernt.

Vorteilhafterweise kann diese lmmunadsorptionsbehandlung stationär und ambulant durchgeführt werden. Es kann vorgesehen sein, dass die Vorrichtung, insbesondere der so genannte Adsorber, Bestandteil eines extrakorporalen Blutkreislaufes ist. Hierbei wird dem Patienten aus einem größeren Körpergefäß, insbesondere einer Armvene, kontinuierlich bzw. diskontinuierlich Blut entnommen und mittels Filtration oder Zentrifugation in einzelne Bestandteile, wie beispielsweise die zellulären und die humoralen Bestandteile, separiert. Ein wesentlicher Bestandteil des Blutes, das hierdurch gewonnen wird, ist insbesondere Blutplasma. Das Blutplasma kann vorteilhafterweise durch die erfindungsgemäße Vorrichtung geleitet und nach Adsorption der Autoantikörper zusammen mit den zuvor separierten Blutbestandteilen, insbesondere den zellulären Bestandteilen, dem Patienten zurückgegeben werden, insbesondere durch eine andere Arm- bzw. Beinvene. Es kann weiterhin vorgesehen sein, dass die Peptide an einer Sepharose-Matrix immobilisiert sind. Diese Matrix kann in einen Behälter gegeben werden, der ein Volumen von 10 bis 400 ml aufweist. Das Blutplasma des Patienten kann dann über diese Matrix geleitet werden, wobei die Autoantikörper binden und so aus dem Blutplasma eliminiert werden können.

Dem Fachmann sind verschiedene Möglichkeiten bekannt, derartige festphasenfixierte Peptide bereitzustellen, beispielsweise in Form von (i) regenerationsfähigen Adsorptionssäulen, in Form. von (ii) Doppelsäulen als auch in Form von (iii) Einmalsäulen. Die verschiedenen Spül- und Elutionslösungen, die eine hohe Effizienz der Behandlung ermöglichen, können durch den Fachmann problemlos durch Routineversuche ermittelt werden. Durch die Bereitstellung der erfindungsgemäßen Lehre, insbesondere der erfindungsgemäßen Peptide, sind dem Fachmann verschiedene Möglichkeiten. offenbart, diese in vivo, ex vivo und in vitro, zur Prophylaxe, Diagnose, Therapie als auch zur Nachbehandlung von Kälteinduzierten, Autoantikörper-vermittelten Krankheiten einzusetzen.

In einer weiteren bevorzugten Ausführungsform der Erfindung wird das Molekül GP2 zur Immunisierung von Säugetieren zur Gewinnung von poly-, monoklonalen oder antiideotypischen Autoantikörpern verwendet.

In einer weiteren bevorzugten Ausführungsform der Erfindung ist das Molekül GP2 ausgewählt aus der Gruppe umfassend:
a) ein Molekül aufweisend eine Aminosäuresequenz, die eine ausreichende Homologie zu dem Molekül GP2 aufweist, um zu diesem funktionsanalog zu sein,
b) ein Molekül nach a), welches durch Deletion, Addition, Substitution, Translokation, Inversion und/oder Insertionen modifiziert wurde und zu dem Molekül gemäß a) funktionsanalog ist.

In einer weiteren bevorzugten Ausführungsform der Erfindung ist das unter b) angegebene Molekül mindestens 40% homolog zu dem unter a) angegebenen Molekül.

In einer weiteren bevorzugten Ausführungsform der Erfindung ist das unter b) angegebene Molekül mindestens 60%, vorzugsweise 70%, bevorzugt 80%, ganz besonders bevorzugt 90% homolog zu dem unter a) angegebenen Molekül.

In einer weiteren bevorzugten Ausführungsform der Erfindung liegt das Molekül GP2 linear oder in der zyklischen Form vor, wobei die Peptidzyklisierung bei Vorhandensein von zwei Zysteinen durch Disulfid-Brückenbindung erfolgt oder durch Amid-Zyklisierung, welche wahlweise über die Seitenketten, über die terminalen C und N oder durch eine Kombination dieser Möglichkeiten erfolgt.

In einer weiteren bevorzugten Ausführungsform der Erfindung ist das festphasengebundene Molekül GP2 an organische, anorganische, synthetische oder gemixte Polymere gebunden, vorzugsweise Agarose, Zellulose, Silica Gel, Polyamide oder Polyvinylalkohole.

Die Erfindung betrifft auch eine pharmazeutische Zusammensetzung umfassend mindestens ein Molekül GP2 ggf. zusammen mit einem pharmazeutisch akzeptablen Träger.

In einer bevorzugten Ausführungsform der Erfindung ist vorgesehen, dass der pharmazeutische Träger ausgewählt ist aus der Gruppe umfassend Füllmittel, Sprengmittel, Bindemittel, Feuchthaltemittel, Streckmittel, Lösungsverzögerer, Resorptionsbeschleuniger, Netzmittel, Absorptionsmittel und/oder Gleitmittel.

Die Erfindung betrifft in einem weiteren Aspekt die Verwendung eines spezifischen Liganden für humane Immunglobuline zur Herstellung einer an diesen Liganden gekoppelten Säule zur Behandlung von entzündlichen Darmerkrankungen, wobei diese Behandlung das Passieren von Plasma eines Patienten über die Säule umfasst, wobei Konditionen gewählt werden, welche eine effektive Bindung des spezifischen Liganden an die Immunglobuline im Plasma des Patienten erlauben, wodurch eine signifikante Menge der Immunglobuline aus dem Plasma des Patienten entfernt werden und das so gewonnene Plasma in den Patienten zurückgeführt wird.

Die Erfindung betrifft auch ein Verfahren zur Behandlung von entzündlichen Darmerkrankungen, welches folgende Schritte umfasst:
a) Bereitstellung einer Säule, an welche spezifische Liganden für humane Immunglobuline gekoppelt sind,
b) Passieren von Plasma des Patienten über die Säule unter Konditionen, welche eine effektive Bindung des spezifischen Liganden an die Immunglobuline im Plasma des Patienten erlauben, wodurch eine signifikante Menge der Immunglobuline aus dem Plasma des Patienten entfernt werden und
c) Rückführung des so gewonnenen Plasmas in den Patienten.

In einem weiteren Aspekt der Erfindung betrifft diese ein Verfahren zur Behandlung von entzündlichen Darmerkrankungen, wobei der spezifische Ligand ausgewählt ist aus der Gruppe bestehend aus polyklonalen anti-humanen Immunglobulinantikörpern, monoklonalen anti-humanen lmmunglobulinantikörpern, Fragmenten dieser Antikörper, rekombinanten Molekülen der Antikörper-Idiotypen, synthetisierten Peptiden, Protein A und/oder Protein B.

In einer bevorzugten Ausführungsform der Erfindung erkennt der spezifische Ligand gegen Darm-Gewebe gerichtete Autoantikörper.

In einer weiteren bevorzugten Ausführungsform der Erfindung ist der spezifische Ligand ein Antigen-imitierendes Molekül, ausgewählt aus der Gruppe bestehend aus polyklonalen und monoklonalen antiideotypischen Antikörpern, Fragmenten dieser oder synthetischen Peptiden.

In einer weiteren bevorzugten Ausführungsform der Erfindung ist der spezifische Ligand ein synthetisiertes Peptid, welches eine Sequenz einer Struktur von GP2 imitiert.

Die Erfindung betrifft auch ein Diagnosetestbesteck (Kit) zur Bestimmung von Autoimmunerkrankungen.

In einem weiteren Aspekt betrifft die Erfindung ein immunogenes Mittel, das mindesten ein Molekül GP2 enthält.

Die anmeldungsgemäße Lehre zeichnet sich durch folgende Merkmale aus:
- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff)
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Diese Eigenschaften betreffen insbesondere die bevorzugten Ausführungsformen der Erfindung.

Im Folgenden soll die Erfindung anhand eines Beispiels näher erläutert werden, ohne auf dieses Beispiel beschränkt zu sein.

### Methoden

### Reinigung von GP2 aus Rattenpankreas

### Gewinnung des Zymogen-Granula ( ZG ) und Reinigung der ZG-Membranen

Alle folgenden Arbeitsschritte sind im Eisbad bzw. unter Kühlung auf 4°C Celsius durchgeführt worden. Das Pankreas von vier ausgewachsenen Wistar-Ratten (ca. 2,4 g Gewebe) wurde mechanisch zerkleinert und im zehnfachen Volumen von eiskalter 0,3 M Saccharose-Lösung im POTTER-Homogenisator aufgeschlossen (2 Hübe bei 1000 U/min und 2 Hübe bei 1300 U/min). Anschließend wurde der Aufschluss über ein Gaze-Tuch filtriert. Durch Zentrifugation bei 500 g für 10 min wurden Zelltrümmer und die Kerne abgetrennt. Aus dem Überstand wurden durch Zentrifugation bei 3000 g für 10 min die Zymogen-Granula (unteres, weißes festes Pellet) und die Mitochondrien (darüber-liegendes, lockeres bräunliches Pellet) abgeschieden. Die Mitochondrien sind mittels Puffer A (10 mM -Morpholinpropansulfonsäure (MOPS), pH 6,8 ) vorsichtig abgeschwämmt und die Zymogen-Granula in 2 ml 0,1 M Natrium-Karbonat Lösung, 1mM Diisopropylfluorophosphat (DFP), mittels Vortexer resuspendiert worden. Die Lyse der Granula erfolgte 1 h im Eisbad. Der Lyse-Ansatz wurde über einen diskontinuierlichen Saccharose-Gradienten (0,3 M / 1 M) geschichtet und bei 200.000 g für 90 min zentrifugiert. Die Membranfraktion sammelte sich als Bande in der Dichte-Grenzschicht. Die Bande wurde abgesaugt und die gewonnene Lösung 0,3 M an Natrium-Bromid eingestellt. Durch Zentrifugation bei 200.000 g für 60 min wurden die Membranen sedimentiert.

### Solubilisierung des GP-2

Das gewonnene Membranpellet wurde in 0,5 ml Puffer B (20 mM Morpholinethansulfonsäure (MES), pH 7,0 ; 80 mM KCI; 45 µg/ml Saponin ) mittels Ultraschall resuspendiert und nach Zusatz von Phosphatidylinosol-spezifischer Phospholipase C (*B*. *cereus* ) durch Inkubation über 1 h bei 37°C GP2 von der Membran abgespalten. Die Membranen wurden durch Zentrifugation (200.000 g, 60 min) pelletiert und der Überstand mit dem löslichen GP-2 durch Ultrafiltration etwa 1 zu 5 konzentriert.

### Enzyme linked immosorbent assay (ELISA) zur Bestimmung von GP2 Antikörpern

Mikrotiterplatten (Maxisorb, Nunc, Roskilde) wurden mit 50µl/Kavität einer Lösung von 10µg/ml Ratten GP2 in Beschichtungspuffer (100 mM Na-Karbonat, pH 9,6), über Nacht bei 4°C beschichtet. Nach einmaligem Waschen der Mikrotiterplatte mit einem Waschpuffer (10 mM Na-Phosphat, 150 mM NaCl, 0,1% Tween20, pH 7,4) wurden die Kavitäten mit 300 µl der Blockierungslösung (Waschpuffer, 1% Rinderserumalbumin (RSA), pH 7,4) für 30 min bei Raumtemperatur (RT) inkubiert. Anschließend wurden die Kavitäten dreimal mit Waschpuffer gewaschen und 50 µl 1/100 in Verdünnungspuffer (10 mM Na-Phosphat, 150 mM NaCl, 1% RSA) verdünnte humane Serumproben für 60 min bei RT pro Kavität inkubiert. Nach dreimaligem Waschen mit Verdünnungspuffer wurden die Kavitäten mit 50 µl Konjugatlösung (anti-human IgG-Peroxidase, Schaf, 1µg/ml, Verdünnungspuffer) befüllt und für 30 min bei RT inkubiert. Anschließend wurden die Kavitäten erneut dreimal gewaschen und 50 µl der Substratlösung (Tetramethylbenzidin) pro Kavität dispensiert. Nach 10 min Inkubation bei RT erfolgte das Stoppen der Substratreaktion durch Zugabe von 50 µl Stopplösung (0,3 M Schwefelsäure). Die Optische Dichte der Lösung in den einzelnen Kavitäten wurde mittels Mikrotiterplattenphotometer bichromatisch bei 450 nm und 620 nm gemessen und Computer gestützt mit dem Softwareprogramm EIAstar ausgewertet.

### Indirekte Immunfluoreszenz (IIF) zur Bestimmung von Pankreasantigen Antikörpern

Für die Bestimmung von Pankreasantigen Antikörpern mittel IIF wurden kommerzielle Affenpankreasschnitte (Euroimmun, Lübeck) verwendet. Die Serumproben wurden mit Verdünnungspuffer 1/40, 1/80 und 1/160 verdünnt. Je Reaktionsfeld des Reagenzträgers wurden 25 µl verdünntes Serum pipettiert und die Objektträger mit den Gewebeschnitten 30 min bei RT inkubiert. Anschließend wurden die Objektträger mit einem Phosphatpuffer für 1 min gewaschen. Je Feld des gereinigten Reagenzträgers sind dann 20 µl markiertes Antiserum (anti-human IgG FITC) pipettiert und mit den Gewebeschnitten auf den Objektträgern für 30 min bei RT inkubiert worden. Nach erneutem Waschen mit Phosphatpuffer für 1 min wurden die Objektträger mit Hilfe eines Eindeckmediums mit einem Deckglas versehen. Die Auswertung der Fluoreszenz erfolgte mit Hilfe eines Fluoreszenzmikroskops.

### Literatur

Bossuyt X. Serologic markers in inflammatory bowel disease. Clin Chem 2006, 52 (2): 171-181.
Fricke H, Birkhofer A, Folwaczny C, Meister W, Scriba PC. Characterization of antigens from the human exocrine pancreatic tissue (Pag) relevant as target antigens for autoantibodies in Crohn's disease. Eur J Clin Invest, 1999, 29: 41-45.
Main J, McKenzie H, Yeaman GR, Kerr MA, Robson D, Pennington CR, Parratt D. Antibody to saccharomyces cerevisiae (bakers' yeast) in Crohn's disease. BMJ, 297: 1105-1106.
Mayet WJ, Press AG, Hermann E, Moll R, Manns M, Ewe K, Meyer zum Büschenfelde KH. Antibodies to cytoskeletal proteins in patients with Crohn's disease. Eur J Clin Invest, 1990, 20: 516-524.
Seibold F, Weber P, Jenss H, Wiedmann K H. Antibodies to a trypsin sensitive pancreatic antigen in chronic inflammatory bowel disease: specific markers for a subgroup of patients with Crohn's disease. Gut 1991, 32: 1192-1197.
Stöcker W, Otte M, Ulrich S, Normann D, Finkbeiner H, Stöcker K, Jantschek G, Scriba PC. Autoimmunity to pancreatic juice in Crohn's disease. Results of an autoantibody screening in patients with chronic inflammatory bowel disease. Scand J Gastroenterol, 1987, 22 (suppl 139), 41-52.

## Patentansprüche

1. Ein Molekül GP2 gemäß der Sequenz SEQ ID Nr. 1 für die Verwendung als Arzneimittel.

2. Verwendung eines Moleküls GP2 nach SEQ ID Nr. 1 zur Prophylaxe, Diagnose, Therapie und/oder Nachbehandlung von Autoimmunerkrankungen.

3. Verwendung nach Anspruch 1,
**dadurch gekennzeichnet, dass**
die Autoimmunerkrankung ausgewählt ist aus der Gruppe der entzündlichen Darmerkrankungen (EDE) und/oder der autoimmunen Lebererkrankungen.

4. Verwendung nach Anspruch 2 oder 3,
**dadurch gekennzeichnet, dass**
die entzündliche Darmerkrankung Morbus Crohn und/oder eine chronische Pankreatitis ist.

5. Verwendung nach einem der vorhergehenden Ansprüche,
**dadurch gekennzeichnet, dass**
die Lebererkrankungen Primär Sklerosierende Cholangitis und/oder Autoimmunenteritiden sind.

6. Verwendung nach einem der vorhergehenden Ansprüche,
**dadurch gekennzeichnet, dass**
das Molekül GP2 einen Linker und/oder Spacer ausgewählt aus der Gruppe α-Aminocarbonsäuren sowie deren Homo- und Heterooligomere; α,ω-Aminocarbon-säuren sowie deren verzweigte Homo- oder Hetero-oligomere;
sonstige Aminosäuren sowie die linearen und verzweigten Homo- oder Heterooligomere; Amino-oligoalkoxy-alkylamine; Maleinimidocarbonsäure-Derivate; Oligomere von Alkylaminen; 4-Alkylphenyl-Derivate; 4-Oligoalkoxyphenyl- oder 4-Oligoalkoxy-phenoxy-Derivate; 4-Oligoalkylmercaptophenyl- oder 4-Oligoalkylmercaptophenoxy-Derivate; 4-Oligoalkyl-aminphenyl- oder 4-Oligoalkylaminyphenoxy-Derivate; (Oligoalkylbenzyl)-phenyl- oder 4-(Oligo-alkylbenzyl)-phenoxy-Derivate sowie 4-(Oligoalkoxy-benzyl)-phenyl- oder 4-(Oligoalkoxybenzyl)-phenoxy-Derivate; Trityl-Derivate; Benzyloxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-Phenoxylalkyl- oder Oligoalkoxy-phonxyalkyl-Derivate; Carbamat-Derivate; Amine; Trialkylsilyl- oder Dialkyl-akoxysilyl-Derivate; Alkyl- oder Aryl-Derivate und/oder Kombinationen davon umfasst.

7. Verwendung nach einem der vorhergehenden Ansprüche,
**dadurch gekennzeichnet, dass**
das Molekül GP2 zur Herstellung eines Arzneimittels zur Behandlung von Autoimmunerkrankungen eingesetzt wird.

8. Verwendung nach einem der vorhergehenden Ansprüche,
**dadurch gekennzeichnet, dass**
das Molekül GP2 löslich oder festphasengebunden zum direkten oder indirekten Autoantikörpernachweis in Körperflüssigkeiten, insbesondere Blut und/oder Serum verwendet wird.

9. Verwendung nach einem der vorhergehenden Ansprüche,
**dadurch gekennzeichnet, dass**
zusätzlich zu dem löslichen oder festphasengebundenen Molekül GP2 und unspezifische Adsorbermoleküle verwendet werden, ausgewählt aus der Gruppe umfassend Protein A, Protein G, Anti-Human-Immunglobuline und/oder L-Tryptophan.

10. Verwendung von dem Molekül GP2 zur Immunisierung von Säugetieren zur Gewinnung poly-, monoklonaler und/oder antiideotypischer Autoantikörper.

11. Verwendung nach einem der vorhergehenden Ansprüche,
**dadurch gekennzeichnet, dass**
das Molekül GP2 ausgewählt ist aus der Gruppe umfassend
a) ein Molekül aufweisend eine Aminosäuresequenz, die eine ausreichende Homologie zu dem Molekül GP2 aufweist, um zu diesem funktionsanalog zu sein,
b) ein Molekül nach a), welches durch Deletion, Addition, Substitution, Translokation, Inversion und/oder Insertionen modifiziert wurde und zu dem Molekül gemäß a) funktionsanalog ist.

12. Verwendung nach einem der vorhergehenden Ansprüche,
**dadurch gekennzeichnet, dass**
das unter b) angegebene Molekül mindestens 40% homolog zu dem unter a) angegebenen Molekül ist.

13. Verwendung nach dem vorhergehenden Anspruch,
**dadurch gekennzeichnet, dass**
das unter b) angegebene Molekül mindestens 60%, vorzugsweise 70%, bevorzugt 80%, ganz besonders bevorzugt 90% homolog zu dem unter a) angegebenen Molekül ist.

14. Verwendung nach einem der vorhergehenden Ansprüche,
**dadurch gekennzeichnet, dass**
das Molekül GP2 linear oder in der zyklischen Form vorliegt, wobei die Peptidzyklisierung bei Vorhandensein von zwei Zysteinen durch Disulfid-Brückenbindung erfolgt oder durch Amid-Zyklisierung, welche wahlweise über die Seitenketten, über die terminalen C und N oder durch eine Kombination dieser Möglichkeiten erfolgt.

15. Verwendung nach einem der vorhergehenden Ansprüche,
**dadurch gekennzeichnet, dass**
das festphasengebundene Molekül GP2 an organische, anorganische, synthetische und/oder gemixte Polymere gebunden ist, vorzugsweise Agarose, Zellulose, Silica Gel, Polyamide und/oder Polyvinylalkohole.

16. Pharmazeutische Zusammensetzung umfassend mindestens ein Molekül GP2 gegebenenfalls zusammen mit einem pharmazeutisch akzeptablen Träger.

17. Pharmazeutisches Mittel nach dem vorhergehenden Anspruch,
**dadurch gekennzeichnet, dass**
der pharmazeutische Träger ausgewählt ist aus der Gruppe umfassend Füllmittel, Sprengmittel, Bindemittel, Feuchthaltemittel, Streckmittel, Lösungsverzögerer, Resorptionsbeschleuniger, Netzmittel, Absorptionsmittel und/oder Gleitmittel.

18. Verwendung eines spezifischen Liganden für humane Immunglobuline zur Herstellung einer an diesen Liganden gekoppelten Säule zur Behandlung von entzündlichen Darmerkrankungen, wobei diese Behandlung das Passieren von Plasma eines Patienten über die Säule umfasst, wobei Konditionen gewählt werden, welche eine effektive Bindung des spezifischen Liganden an die Immunglobuline im Plasma des Patienten erlauben, wodurch eine signifikante Menge der Immunglobuline aus dem Plasma des Patienten entfernt werden und das so gewonnene Plasma in den Patienten zurückgeführt wird.

19. Verfahren zur Behandlung von entzündlichen Darmerkrankungen umfassend folgende Schritte:
a) Bereitstellung einer Säule, an welche spezifische Liganden für humane Immunglobuline gekoppelt sind,
b) Passieren von Plasma des Patienten über die Säule unter Konditionen, welche eine effektive Bindung des spezifischen Liganden an die Immunglobuline im Plasma des Patienten erlauben, wodurch eine signifikante Menge der Immunglobuline aus dem Plasma des Patienten entfernt werden und
c) Rückführung des so gewonnenen Plasmas in den Patienten.

20. Verfahren zur Behandlung von entzündlichen Darmerkrankungen nach dem vorhergehenden Anspruch, wobei der spezifische Ligand ausgewählt ist aus der Gruppe bestehend aus polyklonalen anti-humanen Immunglobulinantikörpern, monoklonalen anti-humanen Immunglobulinantikörpern, Fragmenten dieser Antikörper, rekombinanten Molekülen der Antikörper-Idiotypen, synthetisierten Peptiden, Protein A und/oder Protein B.

21. Verfahren zur Behandlung von entzündlichen Darmerkrankungen nach Anspruch 25, wobei der spezifische Ligand gegen Darm-Gewebe gerichtete Autoantikörper erkennt.

22. Verfahren zur Behandlung von entzündlichen Darmerkrankungen nach dem vorhergehenden Anspruch, wobei der spezifische Ligand ein Antigen-imitierendes Molekül ist, ausgewählt aus der Gruppe bestehend aus polyklonalen und monoklonalen antiideotypischen Antikörpern, Fragmenten dieser Antikörper und/oder synthetisierten Peptiden.

23. Verfahren zur Behandlung von entzündlichen Darmerkrankungen nach dem vorhergehenden Anspruch, wobei der spezifische Ligand ein synthetisiertes Peptid ist, welches eine Sequenz einer Struktur von GP2 imitiert.

24. Diagnosetestbesteck zur Bestimmung von Autoimmunerkrankungen umfassend das Molekül GP2 gegebenenfalls mit einer Anweisung zum Kombinieren der Inhalte des Testbestecks und/oder zur Bereitstellung einer Formulierung.

25. Vorrichtung zur Chromatographie, insbesondere zur Apharese, umfassend das Molekül GP2.

26. Vorrichtung nach dem vorhergehenden Anspruch,
**dadurch gekennzeichnet, dass**
das Molekül GP2 an eine Festphase gebunden ist.

27. Verfahren zur Behandlung einer Autoimmunerkrankung durch die Bindung und/oder Entfernung von Autoantikörpern mittels an eine Festphase gebundenen Molekülen GP2.

28. Testkit zur Bestimmung von Autoimmunantikörpern, insbesondere zum Nachweis von entzündlichen Darmerkrankungen, bevorzugt Morbus Crohn, chronische Pankreatitis und/oder Colitis Ulcerosa.

29. Immunogenes Mittel,
**dadurch gekennzeichnet, dass**
es mindestens ein Molekül GP2 enthält.
